# FASCICULE DE BREVET EUROPEEN

(11) **EP 3 313 907 B1**
(45) Date de publication et mention de la délivrance du brevet: **12.03.2025**
(21) Numéro de dépôt: 16747812.2
(22) Date de dépôt: 23.06.2016
(51) Int. Cl.: C08F 220/18, A61K 8/81

(54) **POLYMERE A TITRE D'AGENT EPAISSISSANT ET SUSPENSIF**
POLYMER ALS VERDICKUNGSMITTEL UND SUSPENSIONSMITTEL
POLYMER AS THICKENER AND SUSPENDING AGENT

(30) Priorité: 23.06.2015 FR 1555751
(43) Date de publication de la demande: 02.05.2018
(73) Titulaire: COATEX, 69730 Genay (FR)
(72) Inventeur: CHAMPAGNE, Clémentine, 69300 Caluire-et-Cuire (FR); BONY, Delphine, 69650 Quincieux (FR); SUAU, Jean-Marc, 69480 Lucenay (FR); KENSICHER, Yves, 69620 Theize (FR); MAGNY, Benoît, 69270 Cailloux Sur Fontaine (FR)
(74) Mandataire: Balmefrezol, Ludovic Francis Pierre
(86) Numéro de dépôt international: PCT/FR2016/051537
(87) Numéro de publication internationale: WO 2016/207554

(56) Documents cités:
- US-A- 4 309 330
- US-A1- 2012 231 056
- US-A1- 2014 124 716

## Description

La présente invention se rapporte à de nouveaux polymères utilisables comme agents modificateurs de rhéologie pour des formulations aqueuses et permettant d'induire à la fois de bonnes propriétés d'épaississement, de clarté et de bonnes performances suspensivantes.

Les agents modificateurs de rhéologie, également appelés agents épaississants ou de viscosité, sont présents dans les compositions nettoyantes, que ce soit dans les compositions de soin ou d'hygiène de la personne, par exemple les compositions cosmétiques, ou dans les compositions d'entretien tels que des produits détergents. Ces agents influencent les propriétés rhéologiques (en particulier la viscosité) et esthétiques (telles que la clarté) de la formulation généralement riche en tensioactifs, ainsi que la capacité à suspendre et à stabiliser des particules au sein de la formulation.

Parmi les agents modificateurs de rhéologie couramment utilisés dans les formulations aqueuses, on peut citer les polymères solubles ou gonflables en milieu alcalin, plus connus sous l'appellation « ASE » (pour « Alkali-Soluble or Swellable Emulsions » en anglais) et les polymères solubles ou gonflables en milieu alcalin et modifiés hydrophobes, plus connus sous l'appellation « HASE » (pour « Hydrophobically modified Alkali-Soluble or Swellable Emulsions » en anglais). Ainsi, les documents tels que US2006/0271563, WO2014/090709 et CN104292378 décrivent des compositions aqueuses intégrant des polymères de ce type à titre d'agents modificateurs de rhéologie. Le document US 4309330 décrit un copolymère en émulsion obtenu à partir d'un dérivé du dicyclopentadiène, d'un monomère insaturé, d'un monomère insaturé hydroxylé et d'un autre monomère à fonction isocyanate, utile pour la préparation de revêtement réticulé. Le document US 2012/0231056 décrit une méthode d'épaississement d'une formulation en modifiant son pH. Cette formulation est obtenue à partir d'un copolymère préparé en émulsion directe et en l'absence de monomère réticulant.

Les formulateurs constamment cherchent à accéder à de nouveaux agents dotés des propriétés susmentionnées et, dans la mesure du possible, améliorées en termes de performances, notamment sur une large gamme de pH.

La présente invention vise précisément à répondre à ces objets. Ainsi, elle vise à proposer de nouveaux agents modificateurs de rhéologie, présentant à la fois de bonnes propriétés en termes d'effet épaississant (viscosité) et permettant de conduire à des formulations présentant de bonnes performances suspensivantes et une clarté élevée (phase continue limpide), et ce pour une large gamme de pH.

Les inventeurs ont découvert qu'il est possible d'accéder à une formulation répondant à l'ensemble de ces critères (viscosité, performances suspensivantes et clarté) en mettant en œuvre à titre d'agent modificateur de rhéologie un polymère spécifique. Plus particulièrement, la présente invention, telle que définie dans les revendications en annexe, concerne, selon un premier de ses aspects, un polymère obtenu par polymérisation radicalaire d'un mélange de monomères comprenant :
- au moins un monomère anionique (a) présentant une fonction vinylique polymérisable;
- au moins un monomère hydrophobe non ionique (b) présentant une fonction vinylique polymérisable; et
- un ou plusieurs monomère(s) réticulant(s) (c) dont au moins un composé de formule (I) : 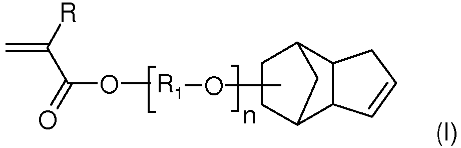 dans laquelle :
   - R est un atome d'hydrogène ou un groupe méthyle,
   - n est 1, et
   - R₁ est un groupe alkyle linéaire ou ramifié en C₁-C₂₀ à l'exception d'un polymère obtenu par polymérisation d'un mélange de monomères comprenant 20 parts en poids d'acide méthacrylique, 10 parts en poids d'acide acrylique, 1 part en poids de composé de formule I dans laquelle R est un groupe méthyle, n est 1, R₁ est un groupe alkyle linéaire en C₂ , 20 parts en poids de méthacrylate de méthyle et 39 parts en poids d'acide acrylique.

De manière avantageuse, comme illustré dans les exemples qui suivent, les polymères selon l'invention procurent à la formulation aqueuse dans laquelle ils sont mis en œuvre de bonnes propriétés suspensivantes, d'épaississement et de clarté, et ce dans une large gamme de pH, c'est-à-dire aussi bien à des valeurs de pH acide, que neutre et basique.

Typiquement, un polymère selon l'invention est obtenu par polymérisation radicalaire d'un mélange de monomères comprenant:
- plus de 20 % en poids par rapport au poids total de monomères formant le polymère d'au moins un monomère anionique (a) présentant une fonction vinylique polymérisable;
- 45 % à 75 % en poids par rapport au poids total de monomères formant le polymère d'au moins un monomère hydrophobe non ionique (b) présentant une fonction vinylique polymérisable; et
- moins de 5 % en poids par rapport au poids total de monomères formant le polymère d'un ou plusieurs monomère(s) réticulant(s) (c) dont au moins un composé de formule (I) tel que défini ci-après.

Par « propriétés suspensivantes » ou « pouvoir suspensif », on entend désigner l'aptitude de la composition à maintenir en suspension des particules dans sa phase continue, en particulier de manière stable dans le temps, par exemple lors du stockage de la composition.

Par « particules » à suspendre, on entend au sens de l'invention désigner des corps solides, pleins ou creux, mais également des entités liquides non miscibles avec la phase continue de la formulation ou encapsulés ou gazeux qui peuvent être caractérisés par des formes, des textures, des structures, des compositions, des couleurs et des propriétés finales différentes. A titre indicatif, on peut citer les particules exfoliantes (par exemple les particules de polyéthylène, les coquilles de fruits pilées, les pierres ponces), les particules nourrissantes (par exemple les sphères de collagène), les particules nacrantes (par exemple le mica titane, les glycols distéarates) et les particules esthétiques (par exemple les bulles d'air, les paillettes, les pigments éventuellement colorés). Pour ce qui est de la suspension de bulles d'air dans la composition, les particules peuvent notamment avoir une taille de 1, 2 ou 3 mm.

Les performances suspensivantes peuvent être évaluées par application d'un « test applicatif suspension » en déterminant notamment la valeur du module d'élasticité G', la valeur de Tan (δ) et la valeur de la résistance élastique, comme décrit dans les exemples qui suivent.

La « clarté » ou « limpidité » de la composition peut être évaluée par mesure de la transmittance de la composition. Une méthode de détermination de la transmittance est décrite dans les exemples qui suivent. Elle est exprimée en pourcentage. Une composition est considérée comme claire ou limpide si elle présente une transmittance, pour une longueur d'onde de 500 nm, d'au moins 60 %, de préférence d'au moins 70 % et plus préférentiellement encore d'au moins 80 %.

D'autres caractéristiques, avantages et modes d'application du polymère selon l'invention ressortiront mieux à la lecture de la description et des exemples qui vont suivre, donnés à titre illustratif et non limitatif.

### DESCRIPTION D'UN POLYMERE SELON L'INVENTION

Comme indiqué précédemment, le polymère selon l'invention obtenu par polymérisation radicalaire d'un mélange de monomères comprend :
- au moins un monomère anionique (a) présentant une fonction vinylique polymérisable;
- au moins un monomère hydrophobe non ionique (b) présentant une fonction vinylique polymérisable; et
- un ou plusieurs monomère(s) réticulant(s) (c) dont au moins un composé de formule (I) : 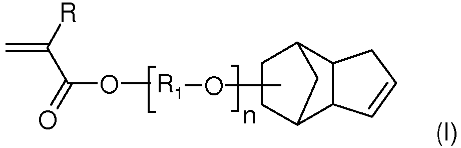 dans laquelle :
   - R est un atome d'hydrogène ou un groupe méthyle,
   - n est 1, et
   - R₁ est un groupe alkyle linéaire ou ramifié en C₁-C₂₀ à l'exception d'un polymère obtenu par polymérisation d'un mélange de monomères comprenant 20 parts en poids d'acide méthacrylique, 10 parts en poids d'acide acrylique, 1 part en poids de composé de formule I dans laquelle R est un groupe méthyle, n est 1, R₁ est un groupe alkyle linéaire en C₂ , 20 parts en poids de méthacrylate de méthyle et 39 parts en poids d'acide acrylique.

De manière préférée selon l'invention, le polymère selon l'invention ne contient pas de monomère hydroxylé à fonction vinylique polymérisable ou bien ne contient pas de monomère à fonction isocyanate.

De manière également préférée selon l'invention, le polymère selon l'invention est obtenu par polymérisation radicalaire d'un mélange de monomères consistant en :
- au moins un monomère anionique (a) présentant une fonction vinylique polymérisable;
- au moins un monomère hydrophobe non ionique (b) présentant une fonction vinylique polymérisable; et
- un ou plusieurs monomère(s) réticulant(s) (c) dont au moins un composé de formule (I) : 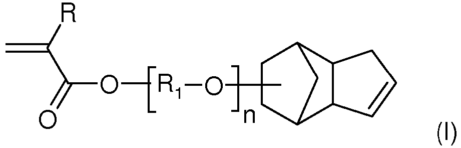 dans laquelle :
   - R est un atome d'hydrogène ou un groupe méthyle,
   - n est 1, et
   - R₁ est un groupe alkyle linéaire ou ramifié en C₁-C₂₀ à l'exception d'un polymère obtenu par polymérisation d'un mélange de monomères comprenant 20 parts en poids d'acide méthacrylique, 10 parts en poids d'acide acrylique, 1 part en poids de composé de formule I dans laquelle R est un groupe méthyle, n est 1, R₁ est un groupe alkyle linéaire en C₂ , 20 parts en poids de méthacrylate de méthyle et 39 parts en poids d'acide acrylique.

Dans la suite du texte, les proportions en monomères entrant dans la composition du polymère selon l'invention sont exprimées en pourcentage en poids par rapport au poids total de monomères mis en œuvre pour former le polymère.

Selon un mode de réalisation particulier, le polymère selon l'invention ne comprend pas d'unité monomérique autre que les monomères (a), (b), et (c) (à l'exception de la présence optionnelle de fragments d'agents de transfert ou d'initiateurs de polymérisation).

Autrement dit, selon une variante de réalisation, la somme des teneurs en monomères (a), (b), et (c) de la composition du polymère selon l'invention est égale à 100 %.

Selon un autre mode de réalisation, le polymère selon l'invention peut comprendre en outre une ou plusieurs unité(s) monomérique(s) additionnelle(s) distincte(s) des monomères (a), (b), et (c).

En particulier, la composition du polymère selon l'invention peut comprendre en outre un ou plusieurs monomère(s) (d) présentant une fonction vinylique polymérisable et une chaîne hydrocarbonée hydrophobe et/ou un ou plusieurs monomère(s) non ionique(s) (e) additionnels comme détaillé plus précisément dans la suite du texte.

Les monomères (a), (b), (c), (d) et (e) de la composition du polymère selon l'invention sont différents. En particulier, le ou lesdits monomères (b) sont différents dudit ou desdits monomères (d) et/ou dudit ou desdits monomères (e).

Selon un mode de réalisation particulier, le polymère selon l'invention est un polymère multiphasique.

Par l'expression « polymère multiphasique », on entend désigner au sens de l'invention une particule multiphasique de polymère, autrement dit une particule de polymère présentant une composition non homogène, préparée par un procédé de polymérisation séquentielle en au moins deux étapes à partir d'au moins deux compositions (ou mélanges) de monomères distinctes.

Comme cela sera exposé ci-après, à l'issue de la première étape, un premier polymère, nommé ci-après polymère P1, est obtenu par polymérisation radicalaire à partir d'un premier mélange de monomères (a), (b) et (c) et éventuellement (d) et/ou (e), puis à la fin de la seconde étape, un second polymère, nommé ci-après polymère P2, est obtenu par polymérisation radicalaire à partir d'un second mélange de monomères (a'), (b') et (c') et éventuellement (d') et/ou (e'). Etant entendu que (a') est un monomère non ionique présentant une fonction vinylique polymérisable, (b') est un monomère hydrophobe non ionique présentant une fonction vinylique polymérisable, (c') est un ou plusieurs monomère(s) réticulant(s) dont éventuellement un composé de formule (I), (d') est un monomère présentant une fonction vinylique polymérisable et une chaine hydrocarbonée hydrophobe au moins en C₁₀ (distinct de (b')), et (e') est un monomère additionnel éventuellement non ionique (distinct de (b')).

Selon ce mode de réalisation particulier, les particules multiphasiques selon l'invention peuvent être notamment structurées en cœur/écorce (ou « core/shell » en anglais), le premier polymère formant le « cœur » et le second polymère formant l'« écorce ». Cette appellation « cœur/écorce » ne doit toutefois pas être interprétée comme désignant une particule dans laquelle la partie « cœur » serait totalement revêtue ou encapsulée par une partie « écorce », mais comme désignant une particule à morphologie contrôlée présentant deux phases distinctes.

Ainsi, au sens de l'invention, l'expression « polymère selon l'invention » comprend aussi bien un polymère obtenu par polymérisation radicalaire d'un seul mélange de monomères tels que définis ci-après, qu'un polymère multiphasique au sens de la présente invention, c'est-à-dire formé de plusieurs compositions polymériques dont au moins une composition polymérique P1 et une composition polymérique P2 comme détaillé dans ce qui suit.

Au sens de la présente invention, on désigne indifféremment l'expression « composition polymérique P1 » et l'expression « polymère P1 ».

Au sens de la présente invention, on désigne indifféremment l'expression « composition polymérique P2 » et l'expression « polymère P2 ».

L'expression « polymère P1 » peut s'entendre d'un unique polymère P1 tel que défini ci-dessus ou de plusieurs polymères P1 obtenus par polymérisation séquentielle.

De même, l'expression « polymère P2 » peut s'entendre d'un unique polymère P2 tel que défini ci-dessus ou de plusieurs polymères P2 obtenus par polymérisation séquentielle.

Selon un mode de réalisation particulier, le polymère P1 ne comprend pas d'unité monomérique autre que les monomères (a), (b), et (c) (à l'exception de la présence optionnelle de fragments d'agents de transfert ou d'initiateurs de polymérisation).

Selon un mode de réalisation particulier, le polymère P2 ne comprend pas d'unité monomérique autre que les monomères (a'), (b'), et (c') (à l'exception de la présence optionnelle de fragments d'agents de transfert ou d'initiateurs de polymérisation).

Autrement dit, selon une variante de réalisation, la somme des teneurs en monomères (a), (b), et (c) de la composition du polymère P1 (respectivement (a'), (b'), et (c') de la composition du polymère P2) est égale à 100 %.

Selon un autre mode de réalisation, le polymère P1 et/ou le polymère P2 peu(ven)t comprendre, en outre, une ou plusieurs unité(s) monomérique(s) additionnelle(s) distincte(s) des monomères (a), (b), et (c) (respectivement (a'), (b'), et (c')).

En particulier, la composition du polymère P1 (respectivement du polymère P2) peut comprendre en outre un ou plusieurs monomère(s) (d) (respectivement (d')) présentant une fonction vinylique polymérisable et une chaîne hydrocarbonée hydrophobe et/ou un ou plusieurs monomère(s) (e) additionnels éventuellement non ionique(s) (respectivement (e')) comme détaillé plus précisément dans la suite du texte.

Par ailleurs, il est entendu que les monomères (a) et (a') (respectivement (b) et (b'), respectivement (c) et (c'), respectivement (d) et (d'), respectivement (e) et (e')) entrant dans la composition du polymère P1 et du polymère P2 peuvent être de même nature dans le polymère P1 et dans le polymère P2, ou de natures différentes.

Les monomères (a), (b), (c), (d) et (e) de la composition du polymère P1 sont différents. En particulier, le ou lesdits monomères (b) sont différents du ou desdits monomères (d) et/ou du ou desdits monomères (e). Il en va de même pour les monomères (a'), (b'), (c'), (d') et (e') de la composition du polymère P2.

Selon un mode de réalisation particulier, la répartition pondérale polymère P1/polymère P2 du polymère multiphasique selon l'invention est comprise entre 45/55 et 95/5, en particulier entre 60/40 et 90/10.

### Monomère anionique présentant une fonction vinylique polymérisable dit « monomère (a) »

Selon un mode de réalisation particulier, les monomères anioniques (a) (et (a')) présentant une fonction vinylique polymérisable, appelés plus simplement dans la suite du texte « monomères anioniques », comprennent au moins un groupement carboxylique.

En particulier, les monomères anioniques peuvent être choisis parmi l'acide acrylique, l'acide méthacrylique, l'acide maléique, l'acide itaconique, l'acide crotonique et leurs mélanges, et/ou les sels de ces acides.

Selon un mode de réalisation particulier, les monomères anioniques peuvent être choisis parmi les monomères d'acide acrylique et/ou d'acide méthacrylique et/ou l'un de leurs sels.

Selon un autre mode de réalisation, les monomères anioniques peuvent être choisis parmi les monomères d'acide acrylique et/ou d'acide méthacrylique.

De préférence, le monomère anionique du polymère selon l'invention est l'acide méthacrylique (AMA).

Le ou lesdits monomères anioniques peuvent représenter plus de 20 % en poids, par exemple au moins 23 % en poids, ou par exemple au moins 25 % en poids, en particulier de 25 % à 50 % en poids, et plus particulièrement de 27 % à 41 % en poids, par rapport au poids total de monomères formant le polymère.

Selon un mode de réalisation particulier, lorsque le polymère selon l'invention est un polymère multiphasique :
- le ou lesdits monomères anioniques (a) peuvent représenter plus de 20 % en poids, par exemple au moins 23 % en poids, ou par exemple au moins 25 % en poids, en particulier de 25 % à 50 % en poids, et plus particulièrement de 27 % à 41 % en poids, par rapport au poids total de monomères formant le polymère P1, et
- le ou lesdits monomères anioniques (a') peuvent représenter plus de 20 % en poids, par exemple au moins 23 % en poids, ou par exemple au moins 25 % en poids, en particulier de 25 % à 50 % en poids, et plus particulièrement de 26 % à 35 % en poids, par rapport au poids total de monomères formant le polymère P2.

Selon un autre mode de réalisation encore, la proportion massique de monomère (a') dans le polymère P2 (teneur massique en monomères (a') par rapport au poids total de monomères formant le polymère P2) est inférieure à la celle dans le polymère P1 (teneur massique en monomères (a) par rapport au poids total de monomères formant le polymère P1).

### Monomère hydrophobe non ionique présentant une fonction vinylique polymérisable dit « monomère (b) »

Les monomères hydrophobes non ioniques (b) (et (b')) présentant une fonction vinylique polymérisable, appelés plus simplement dans la suite du texte « monomères hydrophobes non ioniques », sont des monomères ne présentant ni charge positive ni charge négative en solution aqueuse.

Ils peuvent être choisis parmi les esters, les amides ou les nitriles des acides acryliques ou méthacryliques ou parmi l'acrylonitrile, le styrène, le méthylstyrène, le diisobutylène, la vinylpyrrolidone ou la vinylcaprolactame.

Tout particulièrement, les monomères hydrophobes non ioniques peuvent être choisis parmi les acrylates d'alkyle en C₁-C₈ ou les méthacrylates d'alkyle en C₁-C₈, tels que l'acrylate de méthyle, l'acrylate d'éthyle (également nommé AE dans la suite du texte), l'acrylate de butyle, l'acrylate de 2-éthyle-hexyle, le méthacrylate de méthyle, le méthacrylate d'éthyle, le méthacrylate de butyle et leurs mélanges.

Selon un mode de réalisation particulier, les monomères hydrophobes non ioniques peuvent être choisis parmi l'acrylate de méthyle, l'acrylate d'éthyle, l'acrylate de butyle, le méthacrylate d'éthyle et leurs mélanges.

En particulier, le monomère hydrophobe non ionique du polymère selon l'invention peut être l'acrylate d'éthyle.

Le ou lesdits monomères hydrophobes non ioniques peuvent représenter de 45 % à 75 % en poids, en particulier de 48 % à 68 % en poids et plus particulièrement de 50 % à 64 % en poids, par rapport au poids total de monomères formant le polymère selon l'invention.

Le ou lesdits monomères anioniques et le ou lesdits monomères hydrophobes non ioniques peuvent représenter plus de 83 % en poids, en particulier entre 83 % et 99,8 % ou entre 85 % et 99,6 % en poids de la composition globale du polymère de l'invention.

Par « composition globale », on entend le poids total des monomères mis en œuvre pour la synthèse du polymère selon l'invention.

Selon un mode de réalisation particulier, le polymère selon l'invention est tel que :
- le monomère anionique est choisi parmi l'acide acrylique et/ou l'acide méthacrylique et/ou l'un de leurs sels ; par exemple, il est choisi parmi l'acide acrylique et/ou l'acide méthacrylique ; en particulier ce monomère est l'acide méthacrylique et
- le monomère hydrophobe non ionique est choisi parmi l'acrylate de méthyle, l'acrylate d'éthyle, l'acrylate de butyle, le méthacrylate d'éthyle et leurs mélanges, en particulier ce monomère est l'acrylate d'éthyle.

Selon un mode de réalisation particulier, lorsque le polymère selon l'invention est un polymère multiphasique, le ou lesdits monomères hydrophobes non ioniques (b) peuvent représenter de 45 % à 75 % en poids, en particulier de 48 % à 65 % en poids et plus particulièrement de 50 % à 60 % en poids, par rapport au poids total de monomères formant le polymère P1 et le ou lesdits monomères hydrophobes non ioniques (b') peuvent représenter de 45 % à 75 % en poids, en particulier de 50 % à 68 % en poids et plus particulièrement de 55 % à 64 % en poids, par rapport au poids total de monomères formant le polymère P2.

Selon un mode de réalisation particulièrement préféré, la proportion massique de monomères (b') dans le polymère P2 (teneur massique en monomères (b') par rapport au poids total de monomères formant le polymère P2) est supérieure à celle dans le polymère P1 (teneur massique en monomères (b) par rapport au poids total de monomères formant le polymère P1).

Selon un mode de réalisation, la répartition pondérale monomères hydrophobes non ioniques (b')/monomères anioniques (a') de la composition du polymère P2 est comprise entre 60/40 et 85/15, en particulier entre 65/35 et 80/20.

Selon un mode de réalisation particulier, la répartition pondérale monomères hydrophobes non ioniques (b)/monomères anioniques (a) de la composition du polymère P1 est comprise entre 53/47 et 70/30, en particulier entre 55/45 et 68/32.

### Monomère réticulant dont au moins un composé de formule (I) dit « monomère (c) »

Le mélange de monomères conforme à l'invention comprend en outre un ou plusieurs monomères réticulants (c) dont au moins un composé de formule (I) tel que défini ci-après.

Selon un mode de réalisation particulier, lorsque le polymère selon l'invention est un polymère multiphasique, les mélanges de monomères conduisant au polymère P1 et au polymère P2 comprennent tous les deux en outre un ou plusieurs monomères réticulants respectivement (c) et (c'), un seul des monomères (c) ou (c') comprenant au moins un composé de formule (I) comme défini ci-après. Ainsi selon ce mode de réalisation, soit le mélange de monomères conduisant au polymère P1 soit le mélange de monomères conduisant au polymère P2 comprend un composé de formule (I) comme défini ci-après.

Selon un autre mode de réalisation particulier, lorsque le polymère selon l'invention est un polymère multiphasique, les mélanges de monomères conduisant au polymère P1 et au polymère P2 comprennent tous les deux en outre un ou plusieurs monomères réticulants respectivement (c) et (c'), chacun des monomères (c) et (c') comprenant au moins un composé de formule (I) comme défini ci-après.

Les monomères réticulants (c) et (c'), sont appelés plus simplement dans la suite du texte « monomères réticulants ».

Selon un mode de réalisation particulier, le polymère selon l'invention comporte à titre de monomère réticulant un unique composé de formule (I).

Selon un autre mode de réalisation, il comporte deux monomères réticulants différents dont au moins un composé de formule (I).

Selon encore un autre mode de réalisation, il comporte trois monomères réticulants différents dont au moins un composé de formule (I).

Le ou les monomère(s) réticulant(s) est(sont) utilisé(s) pour générer un polymère sous forme de réseau tridimensionnel.

Le composé de formule (I) et le ou les éventuels monomères réticulants additionnels sont définis ci-après.

### (a) Composé de formule (I)

Comme exposé précédemment, les mélanges de monomères conformes à l'invention comprennent obligatoirement à titre de monomère réticulant au moins un composé de formule (I) : 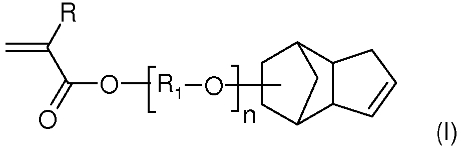 dans laquelle :
- R est un atome d'hydrogène ou un groupe méthyle,
- n est 1, et
- R₁ est un groupe alkyle linéaire ou ramifié en C₁-C₂₀.

Etant entendu que la chaîne ester-éther peut être liée au cycle tricyclo[5.2.1.0^{2,6}] décényle soit par l'atome de carbone du tricycle figurant au-dessus soit par l'atome de carbone du tricycle situé au-dessous de la liaison partant de l'atome d'oxygène du groupe -[R1-O]ₙ-.

Selon un mode de réalisation, le composé de formule (I) est tel que R est un atome d'hydrogène ou un groupe méthyle, R₁ est un groupe -(CH₂)₂-, et n est 1.

Selon un autre mode de réalisation encore, le composé de formule (I) est choisi parmi :
- l'éthylène glycol dicyclopentényl éther méthacrylate (également nommé éthylène glycol tricyclo[5.2.1.0^{2,6}]décène méthacrylate, EGDCPEMA, tel que par exemple le Fancryl FA-512M^{™} ou encore Fancryl FA-512MT^{™} commercialisé par la société Hitachi Chemical),
- l'éthylène glycol dicyclopentényl éther acrylate (également nommé éthylène glycol tricyclo[5.2.1.0^{2,6}]décène acrylate, EGDCPEA tel que par exemple le Fancryl FA-512AS^{™} commercialisé par la société Hitachi Chemical),
- et mélanges, par exemple le mélange de EGDCPEA et de EGDCPEMA. Selon un autre mode de réalisation encore, le composé de formule (I) est le EGDCPEA.

Selon encore un autre de ses aspects, la présente invention concerne l'utilisation d'un monomère de formule (I) : 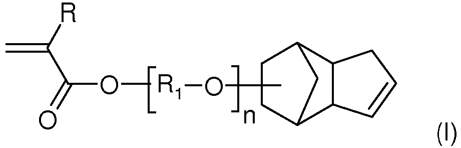 dans laquelle :
- R est un atome d'hydrogène ou un groupe méthyle,
- n est 1 et
- R₁ est un groupe alkyle linéaire ou ramifié en C₁-C₂₀,
en une quantité de moins de 5 % en poids par rapport au poids total de monomères formant le polymère, pour préparer un polymère, en particulier pour préparer un polymère dont la composition est définie ci-dessus.

La présente invention concerne en outre l'utilisation d'un monomère de formule (I) : 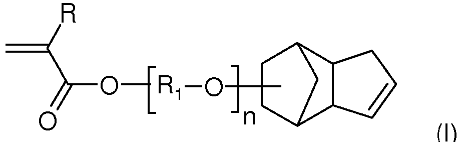 dans laquelle :
- R est un atome d'hydrogène ou un groupe méthyle,
- n est 1, et
- R₁ est un groupe alkyle linéaire ou ramifié en C₁-C₂₀,
en une quantité de moins de 5 % en poids par rapport au poids total de monomères formant le polymère, pour réticuler un polymère/mélange de monomères, en particulier pour réticuler un mélange de monomères tels que mentionnés ci-dessus.

### (b) Monomère(s) réticulant(s) additionnel(s)

Comme indiqué ci-dessus, les mélanges de monomères conformes à l'invention peuvent, outre un composé de formule (I) à titre de monomère réticulant, comprendre également un autre ou plusieurs autres monomère(s) réticulant(s) (c) additionnel(s) distinct(s) du composé de formule (I) tel que défini précédemment.

Selon un mode de réalisation particulier, le polymère selon l'invention comporte un unique autre monomère réticulant distinct du composé de formule (I).

Selon un autre mode de réalisation, il comporte deux monomères réticulants différents, distincts du composé de formule (I).

Selon la présente invention, on utilise en tant que monomère réticulant additionnel distinct d'un composé de formule (I), un monomère qui est un composé polyinsaturé. Ce composé peut comporter deux, trois ou plusieurs insaturations éthylèniques.

Le monomère réticulant additionnel peut avoir un caractère hydrophile, hydrophobe ou amphiphile.

Des exemples de ces composés incluent les composés di(méth)acrylates comme le di(méth)acrylate de polyalkylène glycol, notamment le di(méth)acrylate de polypropylène glycol, le di(méth)acrylate d'éthylène glycol, le di(méth)acrylate de polyéthylène glycol, le di(méth)acrylate de triéthylène glycol, le di(méth)acrylate de 1,3-butylène glycol, le di(méth)acrylate de 1,6-butylène glycol, le di(méth)acrylate de 1,6-hexanediol, le di(méth)acrylate de néopentyl glycol, le di(méth)acrylate de 1,9-nonanediol, mais aussi le 2,2'-bis(4-(acryloxy-propyloxyphényl)propane, le 2,2'-bis(4-(acryloxydiéthoxy-phényl)propane et l'acrylate de zinc ; les composés tri(méth)acrylates tels que le tri(méth)acrylate de triméthylolpropane et le tri(méth)acrylate de triméthylolpropane éthoxylé, le tri(méth)acrylate triméthyloléthane, le tri(méth)acrylate pentaérythritol et le tri(méth)acrylate de tétraméthylolméthane ; les composés tétra(méth)acrylates tels que le tétra(méth)acrylate de ditriméthylolpropane, le tétra(méth)acrylate de tétraméthylolméthane et le tétra(méth)acrylate de pentaérythritol ; les composés hexa(méth)acrylates tels que l'hexa(méth)acrylate de dipentaérythritol ; les composés penta(méth)acrylates tels que le penta(méth)acrylate de dipentaerythritol ; les composés allyls tels que l'allyl (méth)acrylate, le diallylphthalate, l'itaconate de diallyl, le fumarate de diallyl, le maléate de diallyl, et le triallylcyanurate ; les éthers polyallyls du sucrose ayant de 2 à 8 groupes par molécule, les éthers polyallyls du pentaérythritol tels que le pentaérythritol diallyl éther, le pentaérythritol triallyl éther et le pentaérythritol tetraallyl éther ; les éthers polyallyls du triméthylolpropane tels que l'éther diallyl triméthylolpropane et l'éther triallyl triméthylolpropane. D'autres composés polyinsaturés incluent le divinyl glycol, le divinyl benzène, le divinylcyclohexyl et le méthylènebisacrylamide.

Selon un autre aspect, les monomères réticulants additionnels peuvent être préparés par une réaction d'estérification d'un polyol avec un anhydride insaturé tel que l'anhydride maléique ou l'anhydride itaconique ou par une réaction d'addition avec un isocyanate tel que le 3-isopropényl-diméthylbenzène isocyanate.

On peut également utiliser les composés suivants pour obtenir des monomères réticulants additionnels: polyhaloalkanols tels que le 1,3-dichloroisopropanol et le 1,3-dibromoisopropanol ; haloépoxyalkanes tels que l'épichlorohydrine, l'épibromohydrine, le 2-méthyl épichlorohydrine et l'épiiodohydrine ; polyglycidyls éthers tels que le 1,4-butanediol diglycidyl éther, glycérine-1,3-diglycidyl éther, éthylène glycol diglycidyl éther, propylène glycol diglycidyl éther, diéthylène glycol diglycidyl éther, néopentyl glycol diglycidyl éther, polypropylène glycol diglycidyl éther, bisphénol A-épichlorohydrine époxy résine et des mélanges.

Selon un mode de réalisation particulier, les monomères réticulants additionnels mis en œuvre dans le polymère selon l'invention sont choisis parmi les réticulants trifonctionnels.

Il peut s'agir en particulier du tri(méth)acrylate de triméthylolpropane (TMPTA) ou du tri(méth)acrylate de triméthylolpropane éthoxylé (tel que par exemple le TMPTA 3OE).

Selon un mode de réalisation, le mélange de monomères convenant à l'invention comprend en outre à titre de monomère réticulant (c), au moins un monomère différent du composé de formule (I), choisi dans le groupe consistant en le tri(méth)acrylate de triméthylolpropane, le tri(méth)acrylate de triméthylolpropane éthoxylé, le di(méth)acrylate d'éthylène glycol, le méthylènebisacrylamide, le triallylcyanurate, le diallylphtalate, le diallylmaléate et leurs mélanges.

Selon un autre mode de réalisation, le mélange de monomères conformes à l'invention comprend à titre de monomères réticulants deux monomères distincts à savoir le EGDCPEA et le TMPTA.

Selon un autre mode de réalisation encore, le mélange de monomères conformes à l'invention comprend à titre de monomères réticulants deux monomères distincts à savoir le EGDCPEA et le TMPTA 3OE.

Le ou lesdits monomères réticulants peuvent représenter moins de 5 % en poids, en particulier de 0,2 % à 4,5 %, plus particulièrement de 0,25 % à 1,15 % en poids, et encore plus particulièrement de 0,40% à 1,05% en poids, par rapport au poids total de monomères formant le polymère selon l'invention.

### Monomère présentant une fonction vinylique polymérisable et une chaîne hydrocarbonée hydrophobe dit « monomère (d) »

Le mélange de monomères conforme à l'invention peut comprendre en outre au moins un monomère (d) (ou (d')) présentant une fonction vinylique polymérisable et une chaîne hydrocarbonée hydrophobe au moins en C₁₀, de préférence de C₁₂ à C₃₆, de préférence oxyalkylée, distinct du monomère (b) (ou (b') s'il s'agit du monomère (d')).

Ces monomères peuvent être plus particulièrement choisis parmi les monomères de formule (II) suivante :

T-A-Z (II)

dans laquelle :
- T représente une fonction polymérisable permettant la copolymérisation du monomère (d) (ou (d')),
- A représente une chaîne polymérique constituée de :
   - m motifs d'oxyde d'alkylène de formule -CH₂CHR₁O- avec R₁ représentant un groupement alkyle comprenant de 1 à 4 carbones, par exemple un groupement méthyle ou éthyle, et m variant de 0 à 150,
   - p motifs d'oxyde d'alkylène de formule -CH₂CHR₂O- avec R₂ représentant un groupement alkyle comprenant de 1 à 4 carbones, par exemple un groupement méthyle ou éthyle, et p allant de 0 à 150,
   - n motifs d'oxyde d'éthylène avec n variant de 0 à 150, ou de 10, ou 15, à 150, ou de 10, ou 15, à 100, ou de 15 à 50, ou de 15 à 30,
      dans laquelle les motifs d'oxyde d'alkylène de formule -CH₂CHR₁O-, les motifs d'oxyde d'alkylène de formule -CH₂CHR₂O- et les motifs d'oxyde d'éthylène sont en blocs, alternés ou statistiques et
- Z représente une chaîne grasse, saturée ou insaturée, linéaire, ramifiée, cyclique ou polycyclique, d'au moins 10 atomes de carbone, par exemple de C₁₂ à C₃₆, comportant éventuellement un ou plusieurs hétéroatomes tel que par exemple O, S, N ou P.

Selon un mode de réalisation préféré, la somme de m, p et n n'est pas nulle.

Par « unités propoxylées PO » et « unités butoxylées BO », on entend des unités éthoxylées porteuses sur l'un ou l'autre de leurs carbones, d'un radical méthyle ou éthyle respectivement. Une unité éthoxylée est une unité -CH₂-CH₂-O.

Par « chaîne grasse », on entend une chaîne hydrocarbonée aliphatique d'un acide gras, linéaire, ramifiée, cyclique ou polycyclique comprenant au moins 10 atomes de carbone, par exemple de 12 à 36 atomes de carbone, comportant éventuellement un ou plusieurs hétéroatomes tel que par exemple O, S, N ou P.

Selon un mode de réalisation, la chaîne Z est une chaîne ramifiée comportant 16 atomes de carbone.

L'extrémité T représente plus particulièrement un radical contenant une fonction insaturée polymérisable, appartenant au groupe des esters acrylique, méthacrylique, maléique, itaconique, ou crotonique. L'extrémité T peut notamment être choisie parmi les groupements acrylate, méthacrylate, allylique, vinylique, méthacryluréthane et alpha, alpha-diméthyl-m-isopropényl benzyl uréthane.

Selon un mode de réalisation, le monomère (d) ou (d') répond à la formule (III) suivante :

CH₂=C(R₁)-COO-A-Z (III)

dans laquelle :
- R₁ représente H ou CH₃ et
- A et Z ont la même définition que dans la formule (II) ci-dessus.

Selon un mode de réalisation particulier, A dans les formules (II) et (III) précitées représente une chaîne polymérique constituée de 15 à 150, en particulier de 15 à 50 et notamment de 15 à 30 motifs d'oxyde d'éthylène.

A titre d'exemples, le monomère (d) (ou (d')) peut répondre à la formule (II) ou (III) dans laquelle A et Z sont tels que :
- m et p sont nuls, n vaut 25, R₁ représente CH₃, Z est une chaîne ramifiée comportant 16 atomes de carbone, à savoir le 2-hexyl-1-décanyle,
- m et p sont nuls, n vaut 25, R₁ représente CH₃, Z est une chaîne ramifiée comportant 32 atomes de carbone,
- m et p sont nuls, n vaut 25, R₁ représente CH₃, Z est une chaîne linéaire comportant 22 atomes de carbone,
- m et p sont nuls, n vaut 36, R₁ représente CH₃, Z est une chaîne ramifiée comportant 20 atomes de carbone, à savoir le 2-octyl-1-dodécyle, ou
- m et p sont nuls, n vaut 30, R₁ représente CH₃, Z est une chaîne oxo comportant 12 atomes de carbone.

Selon un mode de réalisation particulier, lorsque le polymère selon l'invention est un polymère multiphasique, le ou lesdits monomères (d) peuvent être présents uniquement dans le polymère P1.

Selon un autre mode de réalisation particulier, lorsque le polymère selon l'invention est un polymère multiphasique, le ou lesdits monomères présentant une fonction vinylique polymérisable et une chaîne hydrocarbonée hydrophobe (alors nommés (d')) peuvent être présents uniquement dans le polymère P2.

Alternativement, le ou lesdits monomères (d) et (d') peuvent être présents à la fois dans le polymère P1 et dans le polymère P2 du polymère multiphasique de l'invention.

Le ou lesdits monomères (d), et éventuellement (d'), peuvent représenter de 0 à 20 % en poids, en particulier de 1 à 15 % en poids, et plus particulièrement de 2 à 12 % en poids, par rapport au poids total de monomères formant le polymère selon l'invention.

En particulier, le ou lesdits monomères (d) peuvent être mis en œuvre à raison d'au moins 0,5 % en poids, en particulier de 0,5 à 12 % en poids, par rapport au poids total de monomères formant le polymère P1.

En particulier, le ou lesdits monomères (d') peuvent être mis en œuvre à raison d'au moins 0,5 % en poids, en particulier de 0,5 à 12 % en poids, par rapport au poids total de monomères formant le polymère P2.

### Monomère additionnel éventuellement non ionique dit « monomère (e) »

Le mélange de monomères conformes à l'invention peut comprendre en outre au moins un monomère (e) (ou (e')) additionnel, éventuellement non ionique, distinct du monomère (b) (ou (b') s'il s'agit du monomère (e')).

Ces monomères additionnels éventuellement non ioniques (e) et (e') peuvent être plus particulièrement choisis parmi :
- l'acide 2-acrylamido-2-méthylpropane sulfonique (tel que notamment le produit commercialisé sous la dénomination AMPS^{®} par la société Lubrizol) et ses sels ;
- les télomères insaturés de l'acide acrylique ;
- les monomères de formule (e1) : 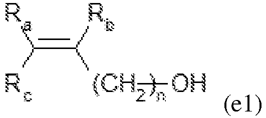 dans laquelle :
   - Rₐ, R_{b} et R_{c} représentent, indépendamment les uns des autres, H ou CH₃, et
   - n est un entier égal à 1 ou à 2 ; et
- les monomères de formule (e2) : 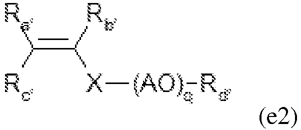 dans laquelle :
   - R_{a'}, R_{b'}, R_{c'} et R_{d'} représentent, indépendamment les uns des autres, H ou CH₃,
   - X représente (C=O) ou (CH₂)ᵣ avec r= 0, 1 ou 2,
   - (AO) représente une chaîne polyalkoxylée constituée d'unités alkoxylées, réparties en blocs, alternées ou statistiques, choisies parmi les unités éthoxylées EO, les unités propoxylées PO, et les unités butoxylées BO ; et
   - q est nul ou représente un nombre entier variant de 1 à 150.

En particulier, les monomères additionnels de formule (e1) peuvent être choisis parmi l'alcool allylique (n=1), l'alcool méthallylique (n=1) et l'isoprénol (n=2). Par exemple, le monomère optionnel est l'isoprénol.

Par « télomères insaturés de l'acide acrylique », on entend des oligomères d'acide acrylique ou d'acide acryloxypropionique, de formule (IV) : 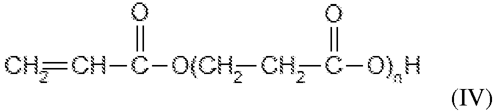 où n est un entier variant de 1 à 10. Ces différents oligomères peuvent être en mélange. Lorsque n = 1, l'oligomère est un dimère d'acide acrylique.

Il est entendu que les différents modes particuliers décrits pour chacun des monomères anioniques, monomères hydrophobes non ioniques, monomères réticulants, monomères présentant une fonction vinylique polymérisable et une chaîne hydrocarbonée hydrophobe et monomères additionnels éventuellement non ioniques du polymère selon l'invention peuvent être combinés.

Le ou lesdits monomères additionnels éventuellement non ioniques (e) et éventuellement (e') peuvent représenter moins de 50 % en poids, en particulier moins de 40 % en poids et plus particulièrement de 1 % à 30 % en poids, par rapport au poids total de monomères formant le polymère selon l'invention.

Selon un mode de réalisation particulier, le polymère selon l'invention est obtenu à partir d'un mélange de monomères comprenant au moins les monomères suivants :
- un ou plusieurs monomères anioniques choisis parmi l'acide acrylique et/ou l'acide méthacrylique et/ou l'un de leurs sels, de préférence l'acide acrylique et/ou l'acide méthacrylique, en particulier l'acide méthacrylique,
- un ou plusieurs monomères hydrophobes non ioniques choisis parmi l'acrylate de méthyle, l'acrylate d'éthyle, l'acrylate de butyle, le méthacrylate d'éthyle et leurs mélanges, en particulier l'acrylate d'éthyle,
- un ou plusieurs monomères réticulants tels que définis précédemment dont au moins un composé de formule (I) choisi parmi l'éthylène glycol dicyclopentényl éther méthacrylate, l'éthylène glycol dicyclopentényl éther acrylate, le dicyclopentényl éther acrylate et leurs mélanges, de préférence le EGDCPEA, le EGDCPEMA et leurs mélanges, en particulier le EGDCPEA,
- éventuellement un ou plusieurs monomères présentant une fonction vinylique polymérisable et une chaîne hydrocarbonée hydrophobe tels que décrits précédemment ; et
- éventuellement un ou plusieurs monomères additionnels éventuellement non ioniques tels que définis précédemment.

### PROCEDE DE PREPARATION D'UN POLYMERE SELON L'INVENTION

Le polymère selon l'invention peut être préparé par des techniques de polymérisation conventionnelles à partir notamment des monomères (a), (b), (c) et optionnellement (d), (e) et d'un ou plusieurs autres monomères réticulants distincts d'un composé de formule (I) tels que définis précédemment.

Selon un mode de réalisation, le polymère selon l'invention peut être obtenu par polymérisation radicalaire en émulsion, dispersion ou solution.

Selon un autre mode de réalisation, le polymère selon l'invention est obtenu par polymérisation radicalaire en émulsion.

La polymérisation est conduite dans des solvants appropriés, en présence d'initiateurs connus.

A titre d'exemple, l'initiateur de polymérisation peut être un sel persulfate, tel que le persulfate d'ammonium.

La polymérisation radicalaire en émulsion peut être réalisée en présence d'au moins un tensioactif et éventuellement d'au moins un agent de transfert de chaînes, permettant de réguler la masse moléculaire des chaînes produites lors de la polymérisation.

Comme tensioactifs susceptibles d'être utilisés, on peut citer :
- les tensioactifs anioniques, tels que par exemple un sel d'acide gras, un sel alkylsulfate (comme le laurylsulfate de sodium), un sel d'alkyléther sulfate (comme le lauryl éther sulfate de sodium), un sel alkylbenzènesulfonate (comme le dodécylbenzènesulfonate de sodium), un sel alkylphosphate ou un sel sulfosuccinate diester, un sel cocoamphoacétate (comme le sodium cocoamphoacétate), un sel cocoamphodiacétate (comme le sodium cocoamphodiacétate), un sel lauroyl glutamate (comme le sodium lauroyl glutamate), un sel cocoyl isethionate (comme le sodium cocoyl isethionate), un sel lauroyl Methyl isethionate (comme le sodium lauroyl methyl isethionate), un sel Methyl cocoyl taurate (comme le sodium methyl cocoyl taurate), un sel Methyl Oleyl taurate (comme le sodium methyl oleyl taurate), un sel Lauroyl Sarcosinate (comme le sodium lauroyl sarcosinate), un sel Laureth 3 sulfosuccinate (comme le sodium laureth 3 sulfosuccinate), un sel cocoyl apple amino acide (comme le sodium cocoyl apple aminate), un sel cocoyl oat amino acide (comme le sodium cocoyl oat aminate).
- les tensioactifs non ioniques, tel que par exemple un polyoxyéthylène alkyléther ou un ester d'acide gras polyoxyéthylène,
- les tensioactifs cationiques, tels que par exemple les halogénures d'alkyl- et/ou aryl-ammoniums quaternaires,
- les tensioactifs zwitterioniques ou amphotères, tels que par exemple les tensioactifs comprenant un groupe bétaïne, et
- leurs mélanges.

Comme agents de transfert de chaîne, on peut citer, avantageusement, les composés mercaptans comprenant au moins quatre atomes de carbone, tels que le butylmercaptan, le n-octylmercaptan, le n-dodécylmercaptan, le tert-dodécylmercaptan.

La polymérisation en émulsion est réalisée, classiquement, dans un milieu de dispersion aqueux.

Ainsi, selon un autre de ses aspects, l'invention concerne un procédé de préparation par polymérisation radicalaire d'un polymère tel que défini précédemment, comprenant au moins l'étape consistant à polymériser un mélange de :
- au moins un monomère anionique (a) présentant une fonction vinylique polymérisable ;
- au moins un monomère hydrophobe non ionique (b) présentant une fonction vinylique polymérisable ;
- un ou plusieurs monomère(s) réticulant(s) (c) dont au moins un composé de formule (I) : 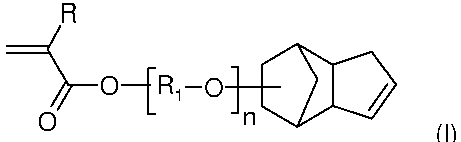 dans laquelle :
   - R est un atome d'hydrogène ou un groupe méthyle,
   - n est 1, et
   - R₁ est un groupe alkyle linéaire ou ramifié en C₁-C₂₀,
- éventuellement au moins un monomère (d) présentant une fonction vinylique polymérisable et une chaîne hydrocarbonée hydrophobe au moins en C₁₀, par exemple de C₁₂ à C₃₆, éventuellement oxyalkylée, distinct du monomère (b), et
- éventuellement au moins un monomère (e) additionnel éventuellement non ionique, distinct du monomère (b), à l'exception d'un mélange de monomères comprenant 20 parts en poids d'acide méthacrylique, 10 parts en poids d'acide acrylique, 1 part en poids de composé de formule I dans laquelle R est un groupe méthyle, n est 1, R₁ est un groupe alkyle linéaire en C₂ , 20 parts en poids de méthacrylate de méthyle et 39 parts en poids d'acide acrylique.

Le polymère de l'invention peut aussi être polymérisé par la voie d'une synthèse en émulsion inverse. Avec cette technique, les monomères sont dissous dans l'eau, le(s) monomère(s) anionique(s) acide(s) étant éventuellement neutralisés en partie ou en totalité. Cette solution des monomères est ensuite émulsionnée dans un solvant comme par exemple un mélange d'alcanes ou une coupe pétrolière, ou un mélange d'huiles synthétiques ou naturelles. La synthèse du polymère est ensuite conduite au moyen d'initiateurs hydrosolubles, permettant la polymérisation au sein de chaque gouttelette en émulsion dans la phase huileuse continue. Cette technique permet en outre d'obtenir des polymères d'une masse moléculaire moyenne plus élevée que par polymérisation en émulsion directe dans l'eau.

Le polymère de l'invention peut aussi être polymérisé par la voie d'une synthèse en phase solvant. Avec cette technique, les monomères sont dissous dans un solvant ou mélange de solvants, comme les solvants chlorés, les solvants aromatiques ou d'autres solvants volatils. La polymérisation est alors conduite au moyen d'initiateurs solubles dans le solvant. Les chaines de polymère vont précipiter lors de leur croissance sous forme d'un solide pulvérulent qui est ensuite séparé par filtration, les solvants résiduels étant ensuite éliminés par évaporation sous vide. Cette technique permet aussi d'obtenir des polymères d'une masse moléculaire moyenne plus élevée que par polymérisation en émulsion directe dans l'eau.

Selon un mode de réalisation particulier, lorsque le polymère selon l'invention est un polymère multiphasique, il peut être préparé de façon séquentielle, par polymérisation radicalaire en émulsion, dispersion ou solution, de préférence en au moins deux étapes consécutives comme explicité ci-après, la première étape étant telle que définie précédemment et permettant l'obtention d'un premier polymère P1.

De préférence, le polymère multiphasique selon l'invention est préparé par polymérisation radicalaire en au moins deux étapes, le polymère P1 et le polymère P2 étant produits dans deux étapes de polymérisation en émulsion séquentielles, en particulier dans cet ordre P1 puis P2.

La polymérisation est conduite dans des conditions appropriées telle que décrites précédemment.

Ainsi, selon un mode de réalisation particulier, un procédé selon l'invention comprend en outre au moins l'étape consécutive suivante:
- polymérisation en présence du polymère P1 précédemment obtenu à l'issue du procédé tel que décrit précédemment, d'un second mélange de monomères permettant d'obtenir un second polymère P2 comprenant :
- au moins un monomère anionique (a') présentant une fonction vinylique polymérisable;
- au moins un monomère hydrophobe non ionique (b') présentant une fonction vinylique polymérisable;
- un ou plusieurs monomère(s) réticulant(s) (c') dont au moins un composé de formule (I) : 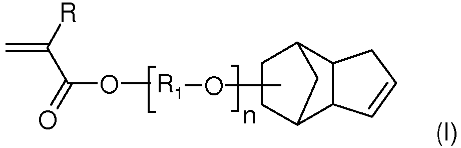 dans laquelle :
   - R est un atome d'hydrogène ou un groupe méthyle,
   - n est 1, et
   - R₁ est un groupe alkyle linéaire ou ramifié en C₁-C₂₀,
- éventuellement au moins un monomère (d') présentant une fonction vinylique polymérisable et une chaîne hydrocarbonée hydrophobe au moins en C₁₀, par exemple de C₁₂ à C₃₆, éventuellement oxyalkylée, distinct du monomère (b'), et
- éventuellement au moins un monomère (e') additionnel éventuellement non ionique, distinct du monomère (b').

D'un point de vue pratique, la première étape consiste à mettre en contact les monomères destinés à entrer dans la composition du polymère P1 avec un initiateur de polymérisation, cette mise en contact pouvant être réalisée en mode discontinu, ou en mode batch, ou en mode semi-batch ou en mode semi-continu (la mise en contact étant réalisée sur une durée pouvant aller de plusieurs minutes à plusieurs heures).

D'un point de vue pratique, la deuxième étape (étape de réalisation du polymère P2) peut se dérouler de la façon suivante :
- une étape d'ajout des monomères destinée à entrer dans la composition du polymère P2 à un milieu de dispersion comprenant le polymère P1 déjà formé, cet ajout pouvant se faire selon un mode discontinu, un mode batch, un mode semi-batch ou un mode semi-continu (la mise en contact étant réalisée sur une durée pouvant aller de plusieurs minutes à plusieurs heures) et
- simultanément pour le mode semi-continu ou postérieurement à cette étape d'ajout pour le mode discontinu, une étape d'introduction d'un initiateur de polymérisation.

### APPLICATIONS D'UN POLYMERE SELON L'INVENTION

Les polymères selon l'invention se révèlent particulièrement efficaces à titre d'agents modificateurs de rhéologie dans une large gamme de compositions aqueuses. On peut citer les compositions aqueuses dans domaines industriels variés et notamment les fluides de fracking en forage, les formulations pour la céramique, les sauces de couchage papetière. On cite en particulier des compositions lavantes renfermant des tensioactifs, telles que des compositions de soin ou des compositions d'entretien (« personal care » et « home care » en anglais). L'expression « compositions de soin » comprend par exemple des compositions cosmétiques, d'hygiène de la personne, des produits de toilette et compositions nettoyantes pour une application sur le corps (incluant la peau, les cheveux, les ongles) des humains ou animaux, par exemple des compositions de shampoing. L'expression « compositions d'entretien » inclut les compositions utilisées pour le nettoyage ou maintien des conditions sanitaires, par exemple dans la cuisine, la salle de bain, les produits détergents, les produits de lessive, etc.

L'invention concerne ainsi, selon encore un autre de ses aspects, une composition aqueuse comprenant au moins un polymère selon l'invention ou tel qu'obtenu selon le procédé décrit ci-avant.

Le polymère selon l'invention peut être mis en œuvre dans la composition aqueuse à raison de 0,1 % à 20 % en poids, en particulier de 0,5 % à 12 % en poids, par rapport au poids total de la composition.

Comme illustré dans les exemples qui suivent, le polymère selon l'invention permet de combiner avantageusement des performances en termes d'effet épaississant, de clarté et de propriétés suspensivantes. Autrement dit, il permet l'obtention d'une composition aqueuse présentant la viscosité souhaitée et comprenant une phase continue limpide et des particules en suspension réparties de manière homogène dans la phase continue.

L'invention concerne ainsi plus particulièrement l'utilisation d'un polymère selon l'invention ou tel qu'obtenu selon un procédé tel que défini précédemment dans une composition aqueuse à titre d'agent épaississant et suspensif.

Ainsi, avantageusement, le polymère selon l'invention peut être utilisé dans des formules riches en tensioactifs à l'image des compositions détergentes et cosmétiques précitées.

Elle concerne encore l'utilisation pour la préparation d'une composition aqueuse stable, comprenant une phase continue limpide et des particules en suspension réparties dans la phase continue, d'un polymère tel que défini précédemment.

L'invention concerne encore un agent pour l'obtention d'une composition aqueuse stable, comprenant une phase continue limpide et des particules en suspension réparties dans la phase continue, comprenant un polymère selon l'invention.

En plus de la clarté qu'il apporte, l'agent de l'invention permet ainsi de maintenir en suspension les particules présentes dans la composition. L'utilisation d'une composition ainsi formulée ne nécessite donc aucune étape de mélange, même si la composition a été stockée plusieurs semaines, voire plusieurs mois.

Une composition selon l'invention peut comprendre des ingrédients classiquement mis en œuvre dans les formulations évoquées précédemment. Elle peut comprendre un ou plusieurs ingrédients actifs (ou agents actifs), sous toute forme que ce soit, et quel que soit le domaine d'application de la composition, comme indiqué précédemment. Le ou les principes actifs peuvent être dissous dans la phase continue de la composition et/ou ils peuvent être sous forme particulaire, non soluble dans la phase continue, et constituer tout ou partie des particules en suspension.

Elle peut comprendre un ou plusieurs tensioactifs, en particulier choisis parmi les tensioactifs anioniques, zwitterioniques ou amphotères, cationiques ou non-ioniques, et leurs mélanges.

L'invention concerne plus particulièrement une composition cosmétique aqueuse, comprenant une phase continue et des particules en suspension dans la phase continue, ladite phase continue et/ou lesdites particules comprenant et/ou consistant en un principe actif cosmétique, ladite composition comprenant un polymère tel que défini précédemment.

A titre de principe(s) actif(s), elle peut comprendre une base lavante pour le corps et/ou les cheveux.

De manière avantageuse, l'agent modificateur de rhéologie selon l'invention permet d'accéder aux propriétés de viscosité, de clarté et d'effet suspensivant souhaitées pour une large de gamme de pH, aussi bien acides, que neutres ou basiques.

Cette large gamme de pH inclut, bien entendu, la valeur de pH moyenne de la peau humaine. L'agent modificateur de rhéologie selon l'invention présente ainsi un intérêt majeur en cosmétique.

L'invention va maintenant être décrite au moyen des exemples suivants, donnés bien entendu à titre illustratif et non limitatif de l'invention.

### EXEMPLES

Les abréviations suivantes sont utilisées :
**AMA** : acide méthacrylique.
**AE** : acrylate d'éthyle.
**MA** : monomère (d) de formule (III) dans laquelle m et p sont nuls, n vaut 25, R₁ représente CH₃, Z est une chaîne ramifiée comportant 16 atomes de carbone, à savoir le 2- hexyldécanyl.
**MA1** : monomère (d) de formule (III) dans laquelle m et p sont nuls, n vaut 25, R₁ représente CH₃, Z est une chaîne ramifiée comportant 32 atomes de carbone, à savoir le 2-tetradécyl-octadécanyl.
**MA2 :** monomère (d) de formule (III) dans laquelle m et p sont nuls, n vaut 25, R₁ représente CH₃, Z est une chaîne linéaire comportant 22 atomes de carbone, à savoir le docosyl,
**MA3 :** monomère (d) de formule (III) dans laquelle m et p sont nuls, n vaut 36, R₁ représente CH₃, Z est une chaîne ramifiée comportant 20 atomes de carbone, à savoir le 2-octyldodécanyl.
**MA4 :** monomère (d) de formule (III) dans laquelle m et p sont nuls, n vaut 30, R₁ représente CH₃, Z est une chaîne oxo comportant 12 atomes de carbone.
**FA-512AS** (commercialisé par la société Hitachi) : éthylène glycol dicyclopentényl éther acrylate (**EGDCPEA**).
**FA-512MT** (commercialisé par la société Hitachi) : éthylène glycol dicyclopentényl éther méthacrylate (**EGDCPEMA**).
**FA-511AAS** (commercialisé par la société Hitachi) : dicyclopentényl éther acrylate (**DCPEA**).
**SR 351** (commercialisé par la société Sartomer) : triacrylate de triméthylolpropane (**TMPTA**).
**SR 454** (commercialisé par la société Sartomer) : triacrylate de triméthylolpropane 3OE (**TMPTA 3OE**).
**TMPDE 90** (commercialisé par la société Perstorp) : triméthylolpropane diallyl éther (**TMPDAE**).
**SR DFM** (commercialisé par la société Sartomer) : TMPDAE monométhacrylé.
**SIPOMER^{®} HPM100** (commercialisé par la société Rhodia) : méthacrylate de nopol 10OE.
**VISIOMER^{®} EGDMA SG** (commercialisé par la société Evonik) : Ethylène Glycol Diméthacrylate (**EDMA**).

### Exemple de synthèse de polymères en procédé semi-batch

Le protocole de synthèse du polymère réalisé en semi batch est le suivant :
On introduit dans un réacteur de 1 L, agité et chauffé à l'aide d'un bain d'huile 432 g d'eau désionisée et 9,29 g d'une solution contenant 28 % massique de lauryl éther sulfate de sodium.

On prépare dans un bécher le pré-mélange comprenant les ingrédients suivants :
- Acrylate d'éthyle : 196,1 g,
- Acide méthacrylique : 99,67 g,
- Macromonomère noté MA : 25,96 g,
- EGDCPEA: 1,38 g,
- Eau désionisée : 172,5 g et
- Solution à 28 % de lauryl éther sulfate de sodium : 6,47 g.

Ce pré-mélange est mis en agitation afin de former une émulsion.

On prépare une solution constituée de 0,1167 g de persulfate de sodium et 5 g d'eau désionisée appelée « initiateur 1 ».

On prépare une solution constituée de 0,3 g de persulfate de sodium et 50 g d'eau désionisée appelée « initiateur 2 ».

On injecte l'initiateur 1 lorsque le réacteur est chauffé à la température de 86°C ± 2°C.

Puis, on injecte dans le réacteur en parallèle la solution d'initiateur 2 de polymérisation durant 2 heures et le pré-mélange de monomères durant 2 heures.

On ajoute alors 35 g d'eau.

On cuit à nouveau une heure à la température de 86°C ± 2°C.

L'ensemble est ensuite refroidi à température ambiante.

### Exemple de synthèse de polymères multiphasiques

Le protocole de synthèse du polymère multiphasique est le suivant :
On introduit dans un réacteur de 1 L, agité et chauffé à l'aide d'un bain d'huile 430 g d'eau désionisée et 9,29 g d'une solution contenant 28 % massique de lauryl éther sulfate de sodium.

On prépare dans un bécher le pré-mélange P1 comprenant les ingrédients suivants :
- Acrylate d'éthyle : 131,74 g,
- Acide méthacrylique : 81,86 g,
- Macromonomère noté MA : 19,82 g,
- EGDCPEA: 1,05 g,
- Eau désionisée : 139,1 g et
- Solution à 28 % de lauryl éther sulfate de sodium : 4,93 g.

Ce pré-mélange est mis sous agitation afin de former une émulsion.

On prépare dans un bécher le pré-mélange P2 comprenant les ingrédients suivants :
- Acrylate d'éthyle : 54,75 g,
- Acide méthacrylique : 26,74 g,
- Macromonomère noté MA : 6,14 g,
- EGDCPEA: 0,33 g,
- Eau désionisée : 42,8 g et
- Solution à 28% de lauryl éther sulfate de sodium : 1,54 g.

Ce pré-mélange est mis sous agitation afin de former une émulsion.

On prépare une solution constituée de 0,318 g de persulfate de sodium et 5 g d'eau désionisée appelée initiateur 1.

On prépare une solution constituée de 0,269 g de persulfate de sodium et 50 g d'eau désionisée appelée initiateur 2.

On injecte l'initiateur 1 lorsque le réacteur est chauffé à la température de 86°C ± 2°C. Puis, on injecte dans le réacteur en parallèle la solution d'initiateur 2 de polymérisation durant 2 heures et le pré-mélange P1 de monomères durant 90 min suivi du pré-mélange P2 en 30 min.

On ajoute alors 35 g d'eau.

On cuit à nouveau une heure à la température de 86°C ± 2°C.

L'ensemble est ensuite refroidi à température ambiante.

L'ensemble des polymères présentés dans les exemples qui suivent ont été synthétisés dans les conditions décrites ci-dessus, en faisant varier les compositions de monomères des pré-mélanges de monomères.

La composition du polymère est indiquée en pourcentage en poids de chacun des monomères par rapport au poids total des monomères formant le polymère.

De manière analogue, lorsqu'il s'agit d'un polymère multiphasique, la composition du polymère P1 et respectivement celle du polymère P2 est indiquée en pourcentage en poids de chacun des monomères par rapport au poids total des monomères de P1 et respectivement de P2).

### Evaluation dans une formulation aqueuse

Les polymères sont testés dans une formulation aqueuse, de composition indiquée dans le tableau 1 suivant (2,4 % ou 3 % en poids de polymère par rapport au poids total de la composition).

**Tableau 1**

| **Composés** | **Quantité (% en poids)** |
|---|---|
| Lauryl éther sulfate de sodium (SLES) | 9 |
| Cocamidopropylbétaïne (CAPB) | 3 |
| Polymère testé | 2,4 ou 3 |
| Eau | Qsp 100 |

Le pH de la formulation est ajusté à une valeur de 5, 6 ou 7 par ajout d'acide lactique ou d'hydroxyde de sodium.

### Propriétés évaluées

Les compositions sont évaluées pour leurs propriétés de clarté, de viscosité et de performances suspensivantes.

### Clarté

La clarté de la composition est évaluée par mesure de la transmittance selon le protocole suivant :
Les mesures sont réalisées sur un Spectromètre UV Genesys 10 UV ^{™} (Cole Parmer), équipé de cuves Rotilabo-Einmal Kuvetten PS, 4,5 mL. De manière pratique, on préchauffe l'appareil 10 minutes avant utilisation. On réalise d'abord une première mesure au moyen d'une cuve remplie de 3,8 mL d'eau bipermutée (le « blanc »). On réalise ensuite la mesure avec une cuve remplie de 3,8 mL de la solution de composition cosmétique à tester. La transmittance est alors mesurée à la longueur d'onde de 500 nm. Plus la valeur de transmittance, exprimée en pourcentage, est élevée plus la composition cosmétique est limpide.

Comme indiqué précédemment, on estime qu'à une valeur de transmittance à 500 nm d'au moins 60 %, la composition est limpide.

### Viscosité

On mesure la viscosité desdites formulations à l'aide d'un viscosimètre Brookfield, modèle LVT. Avant la mesure de la viscosité, on laisse chacune des formulations au repos 24 heures à 25°C. Le mobile doit être centré par rapport à l'ouverture du flacon.
On mesure ensuite la viscosité à 6 rpm (rotations par minute) à l'aide du module approprié. On laisse tourner le viscosimètre jusqu'à ce que la viscosité soit stable.

L'agent modificateur de rhéologie doit apporter une viscosité suffisante à la formulation dans laquelle il est mis en œuvre. D'une manière générale, la viscosité souhaitée pour les formulations épaissies est supérieure à 4 000 mPa.s, en particulier supérieure à 6 000 mPa.s et plus particulièrement supérieure à 8 000 mPa.s.

### Performances suspensivantes

Des mesures de viscoélasticité sont réalisées sur lesdites formulations à l'aide d'un Rhéomètre de type Haake - Mars III. Les variations de Tan(δ) et de G' en fonction de la contrainte (balayage de 0 à 1 000 dyn/cm²) sont mesurées à 25°C à l'aide d'une géométrie cône / plan 1°. Les valeurs de Tan(δ) et de G' à 10 dyn/cm² sont extrapolées et la valeur de résistance élastique est déduite de cette mesure.

En règle générale, la stabilité de particules introduites dans lesdites formulations est observée pour des valeurs combinées de G' > 60 Pa, de Tan(δ) < 0,55 et de résistance élastique > 70 dyn/cm².

### EXEMPLE 1 : Polymères selon l'invention

Les polymères testés nommés pol.1 à pol.20, illustrés dans les tableaux 2 à 6, sont des polymères selon l'invention qui ont été synthétisés selon les protocoles détaillés ci-dessus. Plus précisément, les pol. 1 à pol. 14 sont des polymères préparés selon le procédé en semi-batch tandis que les polymères pol.15 à pol.20 sont des polymères multiphasiques.

En particulier, il est à noter que :
- les pol. 1 et pol.2, figurant dans le tableau 2, sont des polymères ne comprenant pas de monomère (d),
- les pol. 3 à pol.9 et pol. 15 à pol.20, figurant dans les tableaux 2, 3 et 5, sont des polymères comprenant des monomères réticulants (c) divers et
- les pol. 10 à pol.14, figurant dans le tableau 4, sont des polymères comprenant des monomères (d) divers.

**Tableau 2**

| **Polymères testés** | | **Pol. 1** | **Pol. 2** | **Pol.3** | **Pol.4** | **Pol.5** hors invention |
|---|---|---|---|---|---|---|
| **Composition globale** | **AE** | 64,00 | 63,82 | 62,52 | 62,52 | 62,52 |
| | **AMA** | 35,40 | 35,26 | 34,54 | 34,54 | 34,54 |
| | **MA** | - | - | 2,04 | 2,04 | 2,04 |
| | **Réticulant (c)** | 0,60 EGDCPEA | 0,92 EGDCPEA | 0,90 EGDCPEA | 0,90 EGDCPEMA | 0,90 DCPEA |
| **3% actif, pH = 7** | **G' (Pa)** | 97 | 70 | 77 | 91 | 84 |
| | **Tan (δ)** | 0,36 | 0,40 | 0,54 | 0,54 | 0,52 |
| | **Résistance élastique (dyn/cm²)** | 110 | 80 | 110 | 120 | 115 |
| | **T(500nm) (%)** | 98 | 96 | 97 | 98 | 93 |
| | **Visco Brook (mPa.s)** | 17500 | 8700 | 15400 | 19900 | 18200 |
| **3% actif, pH = 6** | **Visco Brook (mPa.s)** | 25300 | 25700 | 29000 | 29000 | 28500 |
| **3% actif, pH=5** | **Visco Brook (mPa.s)** | 12200 | 12300 | 16500 | 16500 | 17600 |

**Tableau 3**

| **Polymères testés** | | **Pol.6** | **Pol.7** | **Pol.8** | **Pol.9** |
|---|---|---|---|---|---|
| **Composition globale** | **AE** | 60,44 | 60,80 | 60,3 | 60,3 |
| | **AMA** | 30,91 | 31,10 | 31,00 | 31,00 |
| | **MA** | 7,80 | 7,80 | 7,80 | 7,80 |
| | **Réticulant (c)** | 0,85 EGDCPEA | 0,30 EGDCPEA | 0,90 EGDCPEA + TMPTA (50/50) | 0,90 EGDCPEA + TMPTA 3OE (50/50) |
| **2,4% actif, pH = 6** | **G' (Pa)** | 120 | 75 | 108 | 126 |
| | **Tan (δ)** | 0,30 | 0,45 | 0,31 | 0,30 |
| | **Résistance élastique (dyn/cm²)** | 115 | 120 | 110 | 120 |
| | **T(500nm) (%)** | 89 | 95 | 90 | 90 |
| | **Visco Brook (mPa.s)** | 17400 | 16400 | 17400 | 19300 |
| **2,4% actif, pH = 5** | **G' (Pa)** | 107 | 66 | 105 | 109 |
| | **Tan (δ)** | 0,30 | 0,44 | 0,31 | 0,30 |
| | **Résistance élastique (dyn/cm²)** | 110 | 90 | 110 | 110 |
| | **T(500nm) (%)** | 85 | 93 | 87 | 87 |
| | **Visco Brook (mPa.s)** | 15700 | 14000 | 15100 | 16300 |

**Tableau 4**

| **Polymères testés** | | **Pol. 10** | **Pol.11** | **Pol. 12** | **Pol. 13** | **Pol.14** |
|---|---|---|---|---|---|---|
| **Composition globale** | **AE** | 60,67 | 60,67 | 60,67 | 60,67 | 60,67 |
| | **AMA** | 31,10 | 31,10 | 31,10 | 31,10 | 31,10 |
| | **Monomère (d)** | 7,80 MA | 7,80 MA1 | 7,80 MA2 | 7,80 MA3 | 7,80 MA4 |
| | **EGDCPEA** | 0,43 | 0,43 | 0,43 | 0,43 | 0,43 |
| **2,4% actif, pH = 6** | **G' (Pa)** | 119 | 109 | 100 | 173 | 92 |
| | **Tan (δ)** | 0,33 | 0,30 | 0,40 | 0,42 | 0,26 |
| | **Résistance élastique (dyn/cm²)** | 150 | 110 | 110 | 300 | 100 |
| | **T(500nm) (%)** | 93 | 80 | 94 | 91 | 91 |
| | **Visco Brook (mPa.s)** | 19200 | 16000 | 21100 | 45100 | 12300 |
| **2,4% actif, pH = 5** | **G' (Pa)** | 97 | 81 | 96 | 149 | 65 |
| | **Tan (δ)** | 0,34 | 0,26 | 0,34 | 0,33 | 0,29 |
| | **Résistance élastique (dyn/cm²)** | 130 | 85 | 105 | 220 | 80 |
| | **T(500nm) (%)** | 91 | 66 | 91 | 83 | 86 |
| | **Visco Brook (mPa.s)** | 16000 | 10300 | 17300 | 32700 | 9800 |

**Tableau 5**

| **Polymères testés** | | **Pol. 15** | **Pol. 16** |
|---|---|---|---|
| **Composition P1** | **AE** | 60,34 | 60,17 |
| | **AMA** | 36,79 | 36,69 |
| | **MA** | 2,17 | 2,17 |
| | **Réticulant (c)** | 0,70 EGDCPEA | 0,97 EGDCPEMA |
| **Composition P2** | **AE** | 67,70 | 68,15 |
| | **AMA** | 29,20 | 29,40 |
| | **MA** | 1,72 | 1,73 |
| | **Réticulant (c)** | 1,38 EGDCPEA | 0,72 EGDCPEMA |
| **Composition globale** | **AE** | 62,52 | 62,52 |
| | **AMA** | 34,54 | 34,54 |
| | **MA** | 2,04 | 2,04 |
| | **Réticulant (c)** | 0,90 | 0,90 |
| **Proportion P1** | | 70,40 | 70,60 |
| **Proportion P2** | | 29,60 | 29,40 |
| **3% actif, pH = 7** | **G' (Pa)** | 83 | 68 |
| | **Tan (δ)** | 0,51 | 0,51 |
| | **Résistance élastique (dyn/cm²)** | 115 | 95 |
| | **T(500nm) (%)** | 97 | 97 |
| | **Visco Brook (mPa.s)** | 17100 | 20400 |
| **3% actif, pH = 6** | **Visco Brook (mPa.s)** | 29700 | 29500 |
| **3% actif, pH = 5** | **Visco Brook (mPa.s)** | 13300 | 14600 |

**Tableau 6**

| **Polymères testés** | | **Pol. 17** | **Pol. 18** | **Pol. 19** | **Pol. 20** hors invention |
|---|---|---|---|---|---|
| **Composition P1** | **AE** | 55,92 | 56,16 | 55,92 | 55,92 |
| | **AMA** | 35,00 | 35,16 | 35,00 | 35,00 |
| | **MA** | 8,19 | 8,23 | 8,19 | 8,19 |
| | **Réticulant (c)** | 0,89 EGDCPEA | 0,45 EGDCPEA | 0,89 EGDCPEMA | 0,89 DCPEA |
| **Composition P2** | **AE** | 62,00 | 62,23 | 62,00 | 62,00 |
| | **AMA** | 30,50 | 30,61 | 30,50 | 30,50 |
| | **MA** | 6,76 | 6,79 | 6,76 | 6,76 |
| | **Réticulant (c)** | 0,74 EGDCPEA | 0,38 EGDCPEA | 0,74 EGDCPEMA | 0,74 DCPEA |
| **Composition globale** | **AE** | 57,58 | 57,82 | 57,58 | 57,58 |
| | **AMA** | 33,77 | 33,92 | 33,77 | 33,77 |
| | **MA** | 7,80 | 7,83 | 7,80 | 7,80 |
| | **Réticulant (c)** | 0,85 | 0,43 | 0,85 | 0,85 |
| **Proportion P1** | | 72,74 | 72,72 | 72,74 | 72,74 |
| **Proportion P2** | | 27,26 | 27,28 | 27,26 | 27,26 |
| **2,4% actif, pH = 6** | **G' (Pa)** | 94 | 141 | 97 | 73 |
| | **Tan (δ)** | 0,29 | 0,28 | 0,30 | 0,47 |
| | **Résistance élastique (dyn/cm²)** | 85 | 150 | 100 | 75 |
| | **T(500nm) (%)** | 78 | 88 | 81 | 94 |
| | **Visco Brook (mPa.s)** | 11800 | 19500 | 13200 | 8700 |
| **2,4% actif, pH = 5** | **G' (Pa)** | 140 | 139 | 144 | 121 |
| | **Tan (δ)** | 0,25 | 0,25 | 0,24 | 0,25 |
| | **Résistance élastique (dyn/cm²)** | 120 | 150 | 130 | 120 |
| | **T(500nm) (%)** | 69 | 80 | 69 | 65 |
| | **Visco Brook (mPa.s)** | 14600 | 18700 | 15300 | 13500 |

Les résultats présentés dans les tableaux 2 à 6 montrent que les polymères selon l'invention possèdent à la fois de bonnes propriétés en termes d'épaississement et permettent d'obtenir des formulations dotées de bonnes performances suspensivantes et de clarté élevée pour l'ensemble des polymères testés.

En outre, les résultats figurant en tableau 3 pour pol.8 et pol.9 montrent qu'il est possible d'utiliser un monomère réticulant (c) additionnel, en l'espèce TMPTA ou TMPTA 3OE, en plus d'un composé de formule (I), en l'espèce EGDCPEA.

### EXEMPLE 2 : Polymères hors invention

Les polymères testés nommés C1 à C9, illustrés dans les tableaux 7 à 10, sont des polymères hors invention qui ont été synthétisés selon les protocoles détaillés ci-dessus et qui comprennent des monomères réticulants non conformes à ceux employés dans la présente invention.

Plus particulièrement, les polymères C1 à C6 sont des polymères préparés selon le procédé semi-batch tandis que les polymères C7 à C9 sont des polymères multiphasiques.

**Tableau 7**

| **Polymères testés** | | **C1** hors invention | **C2** hors invention | **C3** hors invention |
|---|---|---|---|---|
| **Composition globale** | **AE** | 63,54 | 62,52 | 62,52 |
| | **AMA** | 35,56 | 34,54 | 34,54 |
| | **MA** | - | 2,04 | 2,04 |
| | **Réticulant (c)** | 0,90 TMPTA + TMPDAE (75/25) | 0,90 TMPTA | 0,90 TMPTA + TMPDAE Monométhacrylé (63/37) |
| **3% actif, pH = 7** | **G' (Pa)** | 29 | 44 | 49 |
| | **Tan (δ)** | 0,56 | 0,90 | 0,58 |
| | **Résistance élastique (dyn/cm²)** | 30 | 55 | 40 |
| | **T(500nm) (%)** | 92 | 98 | 97 |
| | **Visco Brook (mPa.s)** | 5100 | 14000 | 9100 |
| **3% actif, pH = 6** | **Visco Brook (mPa.s)** | 23600 | 8800 | 24300 |
| **3% actif, pH = 5** | **Visco Brook (mPa.s)** | 14500 | 5300 | 14400 |

**Tableau 8**

| **Polymères testés** | | **C4** hors invention | **C5** hors invention |
|---|---|---|---|
| **Composition globale** | **AE** | 62,52 | 62,52 |
| | **AMA** | 34,54 | 34,54 |
| | **MA** | 2,04 | 2,04 |
| | **Réticulant (c)** | 0,90 5-vinyl-2-norbornene | 0,90 méthacrylate de nopol 10OE |
| **3% actif, pH = 7** | **G' (Pa)** | 52 | 55 |
| | **Tan (δ)** | 1,23 | 1,88 |
| | **Résistance élastique (dyn/cm²)** | 40 | 120 |
| | **T(500nm) (%)** | 95 | 99 |
| | **Visco Brook (mPa.s)** | 3800 | 20100 |
| **3% actif, pH = 6** | **Visco Brook (mPa.s)** | 8220 | 1530 |
| **3% actif, pH = 5** | **Visco Brook (mPa.s)** | 4880 | 2970 |

**Tableau 9**

| **Polymères testés** | | **C6** hors invention |
|---|---|---|
| **Composition globale** | **AE** | 60,44 |
| | **AMA** | 30,91 |
| | **MA** | 7,80 |
| | **Réticulant (c)** | 0,85 TMPTA |
| **2,4% actif, pH = 6** | **G' (Pa)** | 42 |
| | **Tan (δ)** | 0,58 |
| | **Résistance élastique (dyn/cm²)** | 50 |
| | **T(500nm) (%)** | 94 |
| | **Visco Brook (mPa.s)** | 12400 |
| **2,4% actif, pH = 5** | **G' (Pa)** | 32 |
| | **Tan (δ)** | 0,58 |
| | **Résistance élastique (dyn/cm2)** | 40 |
| | **T(500nm) (%)** | 92 |
| | **Visco Brook (mPa.s)** | 10800 |

**Tableau 10**

| **Polymères testés** | | **C7** hors invention | **C8** hors invention | **C9** hors invention |
|---|---|---|---|---|
| **Composition P1** | **AE** | 55,92 | 55,92 | 55,92 |
| | **AMA** | 35,00 | 35,00 | 35,00 |
| | **MA** | 8,19 | 8,19 | 8,19 |
| | **Réticulant (c)** | 0,89 EDMA | 0,89 Tricyclodecane diméthanol diméthacrylate | 0,89 5-vinyl-2-norbornene |
| **Composition P2** | **AE** | 62,00 | 62,00 | 62,00 |
| | **AMA** | 30,50 | 30,50 | 30,50 |
| | **MA** | 6,76 | 6,76 | 6,76 |
| | **Réticulant (c)** | 0,74 EDMA | 0,74 Tricyclodecane diméthanol diméthacrylate | 0,74 5-vinyl-2-norbornene |
| **Composition globale** | **AE** | 57,58 | 57,58 | 57,58 |
| | **AMA** | 33,77 | 33,77 | 33,77 |
| | **MA** | 7,80 | 7,80 | 7,80 |
| | **Réticulant (c)** | 0,85 EDMA | 0,85 Tricyclodecane diméthanol diméthacrylate | 0,85 5-vinyl-2-norbornene |
| **Proportion P1** | | 72,74 | 72,74 | 72,74 |
| **Proportion P2** | | 27,26 | 27,26 | 27,26 |
| **2,4% actif, pH = 6** | **G' (Pa)** | 28 | 26 | Non déterminable |
| | **Tan (δ)** | 0,71 | 0,78 | Non déterminable |
| | **Résistance élastique (dyn/cm²)** | 40 | 40 | Non déterminable |
| | **T(500nm) (%)** | 95 | 97 | 55 |
| | **Visco Brook (mPa.s)** | 7900 | 8100 | 1100 |
| **2,4% actif, pH = 5** | **G' (Pa)** | 28 | 25 | Non déterminable |
| | **Tan (δ)** | 0,65 | 0,68 | Non déterminable |
| | **Résistance élastique (dyn/cm²)** | 40 | 35 | Non déterminable |
| | **T(500nm) (%)** | 94 | 95 | 45 |
| | **Visco Brook (mPa.s)** | 9000 | 8700 | 3100 |

De manière générale, les résultats figurant dans les tableaux 7 à 10 montrent que les propriétés des polymères (effet épaississant, performances suspensivantes et clarté) varient selon la nature du monomère réticulant utilisé non conforme à l'invention.

Certaines comparaisons, à titre d'exemples, sont exposées dans la partie qui suit.

Par exemple, en comparant pol.6 conforme (EGDCPEA) et C6 non conforme (TMPTA), on constate que la formulation comprenant pol.6 possède de meilleures propriétés suspensivantes (valeur de G' significativement plus élevée et valeur de Tan (δ) plus basse), une clarté du même ordre de grandeur et des viscosités globalement plus élevées qu'une formulation comprenant C6 ou TMPTA.

En comparant pol.2 conforme (EGDCPEA) et C1 non conforme (TMPTA + TMPDAE (75/25)), on constate que la formulation comprenant pol.2 possède de meilleures propriétés suspensivantes, ainsi qu'une clarté et des viscosités du même ordre de grandeur à pH 6 et 5 qu'une formulation comprenant C1 ou TMPTA/TMPDAE.

En comparant pol.3, pol.4, pol.5, pol.15 ou pol.16 conformes à C2 ou C3 non conforme, on constate que les formulations comprenant les polymères conformes possèdent de meilleures propriétés suspensivantes et une clarté du même ordre de grandeur.

En comparant pol.3, pol.4, pol.5, pol.15 ou pol.16 conformes à C4 non conforme, on constate que les formulations comprenant les polymères conformes présentent des valeurs de viscosité plus élevées.

En comparant pol.17, pol.19, conformes à C7 ou C8 non conforme, on constate que les formulations comprenant les polymères conformes possèdent de meilleures propriétés suspensivantes, une viscosité plus grande et une clarté du même ordre de grandeur.

Enfin, en comparant pol. 17, pol. 19, ou pol.20 conformes à C9 non conforme, on constate que les formulations comprenant les polymères conformes possèdent une meilleure clarté et une viscosité plus grande.

### EXEMPLE 3 : Gel Douche Ultra-Doux Gommant

Cet exemple illustre l'utilisation d'agents selon l'invention dans des formulations cosmétiques de type gel douche ultra-doux, et a pour but de mettre en évidence les propriétés rhéologiques (suspension et viscosité) et organoleptiques apportées selon l'invention.

Ainsi, à partir d'une formulation de gel douche à base de tensioactifs anioniques et zwitterioniques dont la composition figure dans le tableau 11, le but a consisté à vérifier dans cette formule la limpidité, la viscosité et la suspension telles qu'influencées par différents modificateurs de rhéologie dont ceux faisant référence et ceux selon l'invention.

**Tableau 11**

| | |
|---|---|
| 1-Eau DI (eau bipermutée) | QSP 100 |
| 2-Texapon^{®} NSO UP (BASF) | 32,14 |
| 3-Dehyton^{®} PK 45 (BASF) | 6,67 |
| 4-Modificateur de Rhéologie | **Polymère testé à 3%** |
| 5-Hydroxyde de sodium | Qs pH = 7,0 ± 0,1 |
| 6-Potassium sorbate (Nutrinova) | 0,40 |
| 7-Strawberry Fragrance (Hyteck) | 0,50 |
| 8-Exfoson^{®} Quin 300 red, Particules exfoliantes (Soniam) | 2,00 |

Protocole de préparation de la formulation :
Dans un bécher, on introduit l'eau bipermutée (1), puis sont ajoutés sous agitation les différents ingrédients (2) et (3).
- Après totale homogénéisation, on additionne sous agitation très modérée le modificateur de rhéologie (4).
- On mesure le pH qui est ensuite ajusté à 7,0 ± 0,1 avec l'ingrédient (5).
- Après vérification du pH, le conservateur (6) et le parfum (7) sont mélangés sous agitation modérée à la formulation de gel douche.
- On disperse enfin, sous agitation les particules exfoliantes de quinoa (8).

Le tableau 12 récapitule l'ensemble des modificateurs de rhéologie qui ont été utilisés en tant qu'ingrédient (4) dans le cadre des essais du présent exemple 3.

Dans le tableau 12 :
REF : REFérence / INV : INVention / HINV : Hors INVention.

**Tableau 12**

| | **REF** | **INV** | **HINV** |
|---|---|---|---|
| | NaCl | Pol. 15 | C3 |
| Viscosité Brookfield 6 rpm (mPa.s) | 17800 | 17100 | 9100 |
| Tan (δ) | 12 | 0,51 | 0,58 |
| Résistance élastique (dyn/cm²) | 0 | 115 | 40 |
| T(500 nm) (%) | 98 | 97 | 97 |

## Revendications

1. Polymère obtenu par polymérisation radicalaire d'un mélange de monomères comprenant :
- au moins un monomère anionique (a) présentant une fonction vinylique polymérisable;
- au moins un monomère hydrophobe non ionique (b) présentant une fonction vinylique polymérisable; et
- un ou plusieurs monomère(s) réticulant(s) (c) dont au moins un composé de formule (I) : 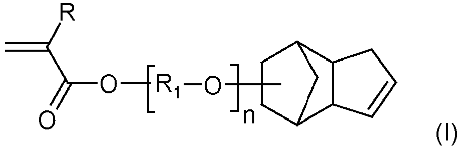 dans laquelle :
- R est un atome d'hydrogène ou un groupe méthyle,
- n est 1, et
- R₁ est un groupe alkyle linéaire ou ramifié en C₁-C₂₀,
à l'exception d'un polymère obtenu par polymérisation d'un mélange de monomères comprenant 20 parts en poids d'acide méthacrylique, 10 parts en poids d'acide acrylique, 1 part en poids de composé de formule I dans laquelle R est un groupe méthyle, n est 1, R₁ est un groupe alkyle linéaire en C₂, 20 parts en poids de méthacrylate de méthyle et 39 parts en poids d'acide acrylique.

2. Polymère selon la revendication 1, dans lequel le composé de formule (I) est tel que R est un atome d'hydrogène ou un groupe méthyle, R₁ est un groupe -(CH₂)₂-, et n est 1.

3. Polymère selon l'une quelconque des revendications précédentes, dans lequel ledit mélange de monomères comprend en outre au moins un monomère (d) présentant une fonction vinylique polymérisable et une chaîne hydrocarbonée hydrophobe au moins en C₁₀, de préférence de C₁₂ à C₃₆, de préférence oxyalkylée, distinct du monomère (b).

4. Polymère selon l'une quelconque des revendications précédentes, dans lequel ledit mélange de monomères comprend en outre au moins un monomère (e) additionnel éventuellement non ionique, distinct du monomère (b).

5. Polymère selon l'une quelconque des revendications précédentes, dans lequel le ou lesdits monomères anioniques (a) sont choisis parmi les monomères d'acide acrylique et/ou d'acide méthacrylique et/ou l'un de leurs sels.

6. Polymère selon l'une quelconque des revendications précédentes, dans lequel le ou lesdits monomères anioniques (a) représentent plus de 20 % en poids, par rapport au poids total de monomères formant le polymère.

7. Polymère selon l'une quelconque des revendications précédentes, dans lequel le ou lesdits monomères hydrophobes non ioniques (b) sont choisis parmi les acrylates d'alkyle en C₁-C₈ ou les méthacrylates d'alkyle en C₁-C₈, tels que l'acrylate de méthyle, l'acrylate d'éthyle, l'acrylate de butyle, l'acrylate de 2-éthyle-hexyle, le méthacrylate de méthyle, le méthacrylate d'éthyle, le méthacrylate de butyle et leurs mélanges ; ou dans lequel le ou lesdits monomères hydrophobes non ioniques (b) représentent de 45 % à 75 % en poids, en particulier de 48 % à 68 % en poids, et plus particulièrement de 50 % à 64 % en poids, par rapport au poids total de monomères formant le polymère.

8. Polymère selon l'une quelconque des revendications précédentes, dans lequel ledit mélange de monomères comprend en outre à titre de monomère réticulant (c), au moins un monomère différent du composé de formule (I), choisi dans le groupe consistant en le tri(méth)acrylate de triméthylolpropane, le tri(méth)acrylate de triméthylolpropane éthoxylé, di(méth)acrylate d'éthylène glycol, méthylènebisacrylamide, triallylcyanurate, diallylphtalate, diallylmaléate et leurs mélanges ; ou dans lequel le ou lesdits monomères réticulants (c) représentent moins de 5 % en poids, par rapport au poids total de monomères formant le polymère.

9. Polymère selon la revendication 3, dans lequel le ou lesdits monomères (d) sont de formule (II) :
T-A-Z (II)
dans laquelle :
- T représente une fonction polymérisable permettant la copolymérisation du monomère (d),
- A représente une chaîne polymérique constituée de :
- m motifs d'oxyde d'alkylène de formule -CH₂CHR₁O- avec R₁ représentant un groupement alkyle comprenant de 1 à 4 carbones, par exemple un groupement méthyle ou éthyle, et m variant de 0 à 150,
- p motifs d'oxyde d'alkylène de formule -CH₂CHR₂O- avec R₂ représentant un groupement alkyle comprenant de 1 à 4 carbones, par exemple un groupement méthyle ou éthyle, et p allant de 0 à 150,
- n motifs d'oxyde d'éthylène avec n variant de 0 à 150, ou de 10, ou 15, à 150, ou de 10, ou 15, à 100, ou de 15 à 50, ou de 15 à 30,
dans laquelle les motifs d'oxyde d'alkylène de formule -CH₂CHR₁O-, les motifs d'oxyde d'alkylène de formule -CH₂CHR₂O- et les motifs d'oxyde d'éthylène sont en blocs, alternés ou statistiques et
- Z représente une chaîne grasse, saturée ou insaturée, linéaire, ramifiée, cyclique ou polycyclique, d'au moins 10 atomes de carbone, comportant éventuellement un ou plusieurs hétéroatomes tel que par exemple O, S, N ou P ; ou dans lequel le ou lesdits monomères (d) représentent de 0 à 20 % en poids, par rapport au poids total de monomères formant le polymère.

10. Polymère selon la revendication 4, dans lequel le monomère additionnel (e) est choisi parmi :
- l'acide 2-acrylamido-2-méthylpropane sulfonique et ses sels,
- les télomères insaturés de l'acide acrylique ;
- les monomères de formule (e1) : 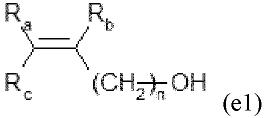 dans laquelle :
- Rₐ, R_{b} et R_{c} représentent, indépendamment les uns des autres, H ou CH₃, et
- n est un entier égal à 1 ou à 2 ; et
- les monomères de formule (e2) : 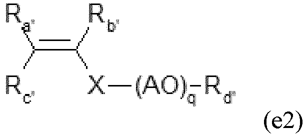 dans laquelle :
- R_{a'}, R_{b'}, R_{c'} et R_{d'} représentent, indépendamment les uns des autres, H ou CH₃,
- X représente (C=O) ou (CH₂)ᵣ avec r= 0, 1 ou 2,
- (AO) représente une chaîne polyalkoxylée constituée d'unités alkoxylées, réparties en blocs, alternées ou statistiques, choisies parmi les unités éthoxylées EO, les unités propoxylées PO, et les unités butoxylées BO ; et
- q est nul ou représente un nombre entier variant de 1 à 150.

11. Procédé de préparation par polymérisation radicalaire d'un polymère tel que défini selon l'une quelconque des revendications 1 à 10, comprenant au moins l'étape consistant à polymériser un mélange de :
- au moins un monomère anionique (a) présentant une fonction vinylique polymérisable ;
- au moins un monomère hydrophobe non ionique (b) présentant une fonction vinylique polymérisable ;
- un ou plusieurs monomère(s) réticulant(s) (c) dont au moins un composé de formule (I) : 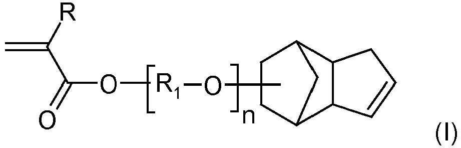 dans laquelle :
- R est un atome d'hydrogène ou un groupe méthyle,
- n est 1, et
- R₁ est un groupe alkylène linéaire ou ramifié en C₁-C₂₀,
- éventuellement au moins un monomère (d) présentant une fonction vinylique polymérisable et une chaîne hydrocarbonée hydrophobe au moins en C₁₀, de préférence de C₁₂ à C₃₆, de préférence oxyalkylée, distinct du monomère (b), et
- éventuellement au moins un monomère (e) additionnel éventuellement non ionique, distinct du monomère (b),
à l'exception d'un mélange de monomères comprenant 20 parts en poids d'acide méthacrylique, 10 parts en poids d'acide acrylique, 1 part en poids de composé de formule I dans laquelle R est un groupe méthyle, n est 1, R¹ est un groupe alkyle linéaire en C₂, 20 parts en poids de méthacrylate de méthyle et 39 parts en poids d'acide acrylique.

12. Procédé selon la revendication 11, comprenant en outre au moins l'étape consécutive suivante :
- polymérisation en présence du polymère P1 précédemment obtenu à l'issue du procédé selon la revendication 11, d'un second mélange de monomères permettant d'obtenir un second polymère P2 comprenant :
- au moins un monomère anionique (a') présentant une fonction vinylique polymérisable;
- au moins un monomère hydrophobe non ionique (b') présentant une fonction vinylique polymérisable;
- un ou plusieurs monomère(s) réticulant(s) (c') dont au moins un composé de formule (I) : 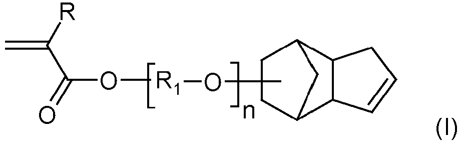 dans laquelle :
- R est un atome d'hydrogène ou un groupe méthyle,
- n est 1, et
- R₁ est un groupe alkylène linéaire ou ramifié en C₁-C₂₀,
- éventuellement au moins un monomère (d') présentant une fonction vinylique polymérisable et une chaîne hydrocarbonée hydrophobe au moins en C₁₀, de préférence de C₁₂ à C₃₆, de préférence oxyalkylée, distinct du monomère (b'), et
- éventuellement au moins un monomère (e') additionnel éventuellement non ionique, distinct du monomère (b').

13. Composition aqueuse comprenant au moins un polymère tel que défini selon l'une quelconque des revendications 1 à 10 ou tel qu'obtenu selon le procédé de la revendication 11 ou 12.

14. Utilisation d'un monomère de formule (I) : 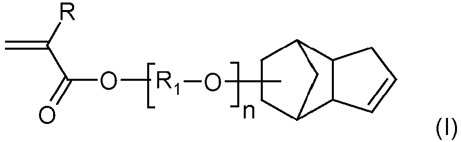 dans laquelle :
- R est un atome d'hydrogène ou un groupe méthyle,
- n est 1, et
- R₁ est un groupe alkylène linéaire ou ramifié en C₁-C₂₀,
en une quantité de moins de 5% en poids par rapport au poids total de monomères formant le polymère, pour préparer un polymère, à l'exception d'un polymère obtenu par polymérisation d'un mélange de monomères comprenant 20 parts en poids d'acide méthacrylique, 10 parts en poids d'acide acrylique, 1 part en poids de composé de formule I dans laquelle R est un groupe méthyle, n est 1, R₁ est un groupe alkyle linéaire en C₂, 20 parts en poids de méthacrylate de méthyle et 39 parts en poids d'acide acrylique.

## Patentansprüche

1. Polymer, das durch radikalische Polymerisation einer Mischung von Monomeren gewonnen wird, umfassend:
- mindestens ein anionisches Monomer (a) mit einer polymerisierbaren funktionellen Vinylgruppe;
mindestens ein nicht-ionisches hydrophobes Monomer (b) mit einer polymerisierbaren funktionellen Vinylgruppe; und
- ein oder mehrere vernetzende Monomere (c), von denen mindestens eine Verbindung die Formel (I) aufweist: 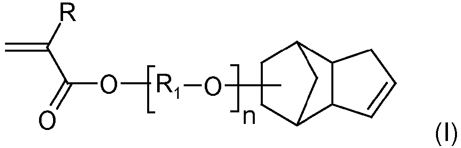 wobei:
- R ein Wasserstoffatom oder eine Methylgruppe ist,
- n 1 ist, und
- R₁ eine lineare oder verzweigte Alkylgruppe mit C₁-C₂₀ ist,
mit Ausnahme eines Polymers, das durch Polymerisation einer Mischung von Monomeren gewonnen wird, das 20 Gew.-Teile Methacrylsäure, 10 Gew.-Teile Acrylsäure, 1 Gew.-Teil einer Verbindung der Formel I umfasst, wobei R eine Methylgruppe ist, n 1 ist und R₁ eine lineare C₂-Alkylgruppe ist, bestehend aus 20 Gew.-Teile Methylmethacrylat und 39 Gew.-Teile Acrylsäure.

2. Polymer nach Anspruch 1, wobei die Verbindung der Formel (I) so beschaffen ist, dass R ein Wasserstoffatom oder eine Methylgruppe, R₁ eine -(CH₂)₂--Gruppe ist und n 1 ist.

3. Polymer nach einem der vorstehenden Ansprüche, wobei die genannte Monomermischung mindestens ein Monomer (d) mit einer polymerisierbaren Vinylfunktion und einer hydrophoben Kohlenwasserstoffkette von mindestens C₁₀, vorzugsweise C₁₂ bis C₃₆, bevorzugt oxyalkyliert, umfasst, das mit Monomer (b) nicht identisch ist.

4. Polymer nach einem der vorstehenden Ansprüche, wobei die genannte Monomermischung ferner mindestens ein weiteres, gegebenenfalls nichtionisches Monomer (e) enthält, das mit Monomer (b) nicht identisch ist.

5. Polymer nach einem beliebigen der vorstehenden Ansprüche, wobei dieses oder diese anionische(n) Monomer(e) (a) ausgewählt ist(sind) aus den Acrylsäure- und/oder Methacrylsäuremonomeren und/oder einem ihrer Salze.

6. Polymer nach einem beliebigen der vorstehenden Ansprüche, wobei dieses oder diese anionische(n) Monomer(e) (a) 20 Gew.-% bezogen auf das Gesamtgewicht an Monomeren, die das Polymer bilden, entspricht bzw. entsprechen.

7. Polymer nach einem der vorstehenden Ansprüche, wobei das oder die nichtionischen hydrophoben Monomere (b) ausgewählt sind aus Alkylacrylaten mit C₁-C₈ oder Alkylmethacrylaten mit C₁-C₈, wie Methylacrylat, Ethylacrylat, Butylacrylat, 2-Ethylhexylacrylat, Methylmethacrylat, Ethylmethacrylat, Butylmethacrylat und deren Mischungen; oder wobei die nichtionischen hydrophoben Monomere (b) 45 bis 75 Gew.-% entsprechen, insbesondere 48 bis 68 Gew.-%, insbesondere 50 bis 64 Gew.-%, bezogen auf das Gesamtgewicht der Monomere, die das Polymer bilden.

8. Polymer nach einem der vorstehenden Ansprüche, wobei die genannte Monomermischung ferner mindestens ein vernetzendes Monomer (c) umfasst, das mit der Verbindung der Formel (I) nicht identisch ist und aus der Gruppe ausgewählt ist, bestehend aus Trimethylolpropantri(meth)acrylat,,ethoxyliertem Trimethylolpropantriacrylat, Di(meth)acrylat von Ethylenglykol, Methylenbisacrylamid, Triallylcyanurat, Diallylphthalat, Diallylmaleat und deren Mischungen; oder wobei die vernetzenden Monomere (c) weniger als 5 Gew.-% entsprechen, bezogen auf das Gesamtgewicht der Monomere, die das Polymer bilden.

9. Polymer nach Anspruch 3, wobei die genannten Monomere (d) die Formel (II) aufweisen:
T-A-Z (II)
wobei:
- T eine polymerisierbare Funktion darstellt, die die Copolymerisation des Monomers (d) ermöglicht,
- A eine Polymerkette darstellt, bestehend aus:
m Alkylenoxideinheiten mit der Formel -CH₂CHR₁O-, wobei R₁ eine Alkylgruppe mit 1 bis 4 Kohlenstoffatomen, zum Beispiel eine Methyl- oder Ethylgruppe ist und m zwischen 0 und 150 variiert,
p Alkylenoxideinheiten mit der Formel - CH₂CHR₂O-, wobei R[g1]2[/g1] eine Alkylgruppe mit 1 bis 4 Kohlenstoffatomen, zum Beispiel eine Methyl- oder Ethylgruppe ist und p zwischen 0 und 150 variiert,
- n Ethylenoxideinheiten, wobei n von 0 bis 150, von 10 oder 15 bis 150, von 10 oder 15 bis 100, von 15 bis 50 oder von 15 bis 30 variiert,
bei der die Alkylenoxideinheiten mit der -CH₂CHR₁O-,, die Alkylenoxideinheiten mit der Formel -CH₂CHR₂O- und die Ethylenoxideinheiten als Blocks, alternierend oder statistisch angeordnet sind;
- Z einer gesättigten oder ungesättigten, linearen, verzweigten, zyklischen oder polyzyklischen Fettsäurekette mit mindestens 10 Kohlenstoffatomen entspricht, die gegebenenfalls ein oder mehrere Heteroatome wie z. B. O, S, N oder P umfasst; oder wobei die Monomere (d) 0 bis 20 Gew.-% des Gesamtgewichts der Monomere, die das Polymer bildenden, entsprechen.

10. Polymer nach Anspruch 4, wobei das zusätzliche Monomer (e) ausgewählt ist aus: 2-Acrylamido-2-Methylpropansulfonsäure und einem ihrer Salze;
- den ungesättigten Telomeren von Acrylsäure;
- den Monomeren mit der Formel (e1): 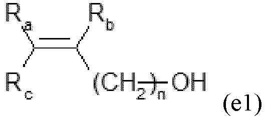 wobei:
- Rₐ, R_{b} und R_{c} unabhängig voneinander H oder CH₃ entsprechen, und
- n einer Ganzzahl 1 oder 2 entspricht; und
- den Monomeren mit der Formel (e2): 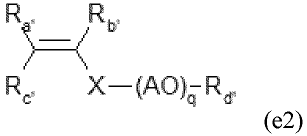 wobei:
- R_{a'}, R_{b'}, R_{c'} und R_{d'} unabhängig voneinander H oder CH₃ entsprechen,
- X (C=O) oder (CH₂)ᵣ mit r= 0, 1 oder 2 entspricht,
- (AO) einer polyalkoxylierten Kette aus alkoxylierten Gruppen entspricht, die in alternierende oder statistische Blöcke aufgeteilt sind, die ausgewählt sind aus ethoxylierten EO-Gruppen, propoxylierten PO-Gruppen und butoxylierten BO-Gruppen; und
- q für 0 oder eine Ganzzahl zwischen 1 und 150 steht.

11. Verfahren zur Herstellung eines Polymers nach einem der Ansprüche 1 bis 10 durch radikalische Polymerisation, umfassend mindestens den Schritt der Polymerisation einer Mischung von:
- mindestens einem anionischen Monomer (a) mit einer polymerisierbaren funktionellen Vinylgruppe;
mindestens einem nicht-ionischen hydrophoben Monomer (b) mit einer polymerisierbaren funktionellen Vinylgruppe;
- einem oder mehreren vernetzenden Monomeren (c), von denen mindestens eine Verbindung die Formel (I) aufweist: 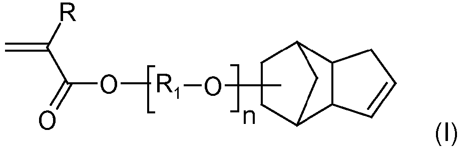 wobei:
- R ein Wasserstoffatom oder eine Methylgruppe ist,
- n 1 ist, und
- R₁ eine lineare oder verzweigte Alkylengruppe mit C₁-C₂₀ ist,
- gegebenenfalls mindestens einem Monomer (d) mit einer polymerisierbaren Vinylfunktion und einer hydrophoben Kohlenwasserstoffkette von mindestens C₁₀, vorzugsweise C₁₂ bis C₃₆, bevorzugt oxyalkyliert, das mit Monomer (b) nicht identisch ist, und
- gegebenenfalls mindestens einem zusätzlichen gegebenenfalls nicht-ionischen Monomer (e), das mit Monomer (b) nicht identisch ist,
mit Ausnahme einer Mischung von Monomeren, die 20 Gew.-Teile Methacrylsäure, 10 Gew.-Teile Acrylsäure, 1 Gew.-Teil einer Verbindung der Formel I enthält, wobei R eine Methylgruppe ist, n 1 ist und R¹ eine lineare Alkylgruppe mit C₂ ist, 20 Gew.-Teilen Methylmethacrylat und 39 Gew.-Teilen% Acrylsäure umfasst.

12. Verfahren nach Anspruch 11, das ferner mindestens den folgenden aufeinanderfolgenden Schritt umfasst:
- Polymerisation in Gegenwart des zuvor nach dem Verfahren nach Anspruch 11 hergestellten Polymers P1, einer zweiten Mischung von Monomeren, mit der ein zweites Polymer P2 hergestellt wird, umfassend:
- mindestens ein anionisches Monomer (a') mit einer polymerisierbaren funktionellen Vinylgruppe;
mindestens ein nicht-ionisches hydrophobes Monomer (b') mit einer polymerisierbaren funktionellen Vinylgruppe;
- ein oder mehrere vernetzende Monomere (c'), von denen mindestens eines Verbindung die Formel (I) aufweist: 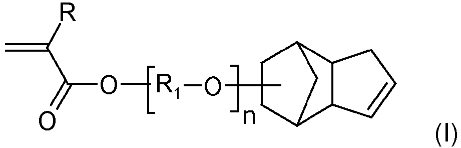 wobei:
- R ein Wasserstoffatom oder eine Methylgruppe ist,
- n 1 ist, und
- R₁ eine lineare oder verzweigte Alkylengruppe mit C₁-C₂₀ ist,
- gegebenenfalls mindestens ein Monomer (d') mit einer polymerisierbaren Vinylfunktion und einer hydrophoben Kohlenwasserstoffkette von mindestens C₁₀,, vorzugsweise C₁₂ bis C₃₆,, bevorzugt oxyalkyliert, das mit Monomer (b') nicht identisch ist, und
- gegebenenfalls mindestens einem zusätzlichen gegebenenfalls nicht-ionischen Monomer (e'), das mit Monomer (b') nicht identisch ist,

13. Wässrige Zusammensetzung aus mindestens einem mehrphasigen Polymer nach einem beliebigen der vorstehenden Ansprüche 1 bis 10 oder nach dem Verfahren aus Anspruch 11 oder 12.

14. Verwendung mindestens eines Monomers mit der Formel (I): 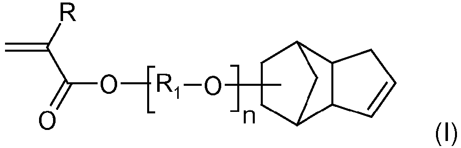 wobei:
- R ein Wasserstoffatom oder eine Methylgruppe ist,
- n 1 ist, und
- R₁ eine lineare oder verzweigte Alkylengruppe mit C₁-C₂₀ ist,
in einer Menge von weniger als 5 Gew.-Teilen bezogen auf das Gesamtgewicht der das Polymer bildenden Monomere, um ein Polymer herzustellen, mit Ausnahme eines Polymers, das durch Polymerisation einer Mischung von Monomeren gewonnen wird, die 20 Gew.-Teile Methacrylsäure, 10 Gew.-Teile Acrylsäure, 1 Gew.-Teil einer Verbindung der Formel I umfasst, wobei R eine Methylgruppe ist, n 1 ist, R₁ eine lineare C₂-Alkylgruppe ist, 20 Gew.-Teile Methylmethacrylat und 39 Gew.-Teile Acrylsäure umfasst.

## Claims

1. A polymer obtained by radical polymerization of a mixture of monomers comprising:
- at least one anionic monomer (a) having a polymerizable vinyl group,
- at least one nonionic hydrophobic monomer (b) having a polymerizable vinyl group and
- one or several cross-linking monomer(s) (c) including at least one compound of formula (I): 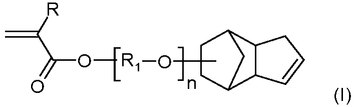 in which:
- R is a hydrogen atom or a methyl group,
- n is equal to 1 and
- R₁ is a C₁-C₂₀ linear or branched alkyl group,
with the exception of a polymer obtained by polymerization of a mixture of monomers comprising 20 parts by weight of methacrylic acid, 10 parts by weight of acrylic acid, 1 part by weight of compound of formula I in which R is a methyl group, n is equal to 1, R₁ is a C₂ linear alkyl group, 20 parts by weight of methyl methacrylate and 39 parts by weight of acrylic acid.

2. The polymer according to claim 1, in which the compound of formula (I) is such that R is a hydrogen atom or a methyl group, R₁ is a -(CH₂)₂- group and n is equal to 1.

3. The polymer according to any one of the preceding claims, in which said mixture of monomers further comprises at least one monomer (d) having a polymerizable vinyl group and an at least C₁₀, preferably C₁₂ to C₃₆, hydrophobic hydrocarbon chain, which is preferably oxyalkylated, distinct from the monomer (b).

4. The polymer according to any one of the preceding claims, in which said mixture of monomers further comprises at least one additional monomer (e) that is optionally nonionic, distinct from the monomer (b).

5. The polymer according to any one of the preceding claims, in which said anionic monomer(s) (a) are chosen among the monomers of acrylic acid and/or of methacrylic acid and/or one of their salts.

6. The polymer according to any one of the preceding claims, in which said anionic monomer(s) (a) represent more than 20% by weight, based on the total weight of monomers forming the polymer.

7. The polymer according to any one of the preceding claims, in which said nonionic hydrophobic monomer(s) (b) are chosen among C₁-C₈ alkyl acrylates or C₁-C₈ alkyl methacrylates such as methyl acrylate, ethyl acrylate, butyl acrylate, 2-ethyl-hexyl acrylate, methyl methacrylate, ethyl methacrylate, butyl methacrylate and mixtures thereof ; or in which said nonionic hydrophobic monomer(s) (b) represent from 45% to 75% by weight, in particular from 48% to 68% by weight and more particularly from 50% to 64% by weight, based on the total weight of monomers forming the polymer.

8. The polymer according to any one of the preceding claims, in which said mixture of monomers further comprises as cross-linking monomer (c), at least one monomer different from the compound of formula (I), chosen in the group consisting of trimethylolpropane tri(meth)acrylate, ethoxylated trimethylolpropane tri(meth)acrylate, ethylene glycol di(meth)acrylate, methylenebisacrylamide, triallylcyanurate, diallylphtalate, diallylmaleate and mixtures thereof ; or in which said cross-linking monomer(s) (c) represent less than 5% by weight, based on the total weight of monomers forming the polymer.

9. The polymer according to claim 3, in which said monomer(s) (d) are of formula (II):
T-A-Z (II)
in which:
- T represents a polymerizable group allowing the copolymerization of the monomer (d),
- A represents a polymeric chain constituted of:
- m units of alkylene oxide of formula -CH₂CHR₁O- with R₁ representing an alkyl group comprising from 1 to 4 carbons, for example an ethyl or methyl group, and m varying from 0 to 150,
- p units of alkylene oxide of formula -CH₂CHR₂O- with R₂ representing an alkyl group comprising from 1 to 4 carbons, for example an ethyl or methyl group, and p ranging from 0 to 150,
- n units of ethylene oxide with n varying from 0 to 150, or from 10 or 15 to 150, or from 10 or 15 to 100, or from 15 to 50, or from 15 to 30,
in which the units of alkylene oxide of formula -CH₂CHR₁O-, the units of alkylene oxide of formula -CH₂CHR₂O- and the units of ethylene oxide are distributed in blocks, alternating or random and
- Z represents a saturated or unsaturated, linear, branched, cyclic or polycyclic, fatty chain of at least 10 carbon atoms, optionally comprising one or several heteroatom(s) such as for example O, S, N or P ; or in which said monomer(s) (d) represent from 0 to 20% by weight, based on the total weight of monomers forming the polymer.

10. The polymer according to claim 4, in which the additional monomer (e) is chosen among:
- the 2-acrylamido-2-methylpropane sulfonic acid and its salts,
- the unsaturated telomers of acrylic acid,
- the monomers of formula (e1): 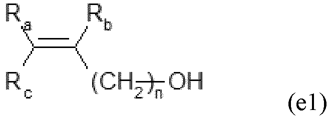 in which:
- Rₐ, R_{b} and R_{c} represent, independently of one another, H or CH₃ and
- n is an integer equal to 1 or to 2 and
- the monomers of formula (e2): 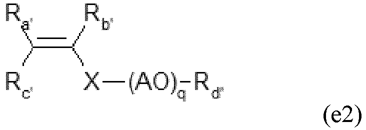 in which:
- R_{a'}, R_{b'}, R_{c'} and R_{d'} represent, independently of one another, H or CH₃,
- X represents (C=O) or (CH₂)ᵣ with r = 0, 1 or 2,
- (AO) represents a polyalkoxylated chain constituted of alkoxylated units, distributed in blocks, alternating or random, chosen among the ethoxylated units EO, the propoxylated units PO and the butoxylated units BO and
- q is equal to 0 or represents an integer varying from 1 to 150.

11. A method for the preparation, by radical polymerization, of a polymer as defined according to any one of claims 1 to 10, comprising at least the step consisting of polymerizing a mixture of:
- at least one anionic monomer (a) having a polymerizable vinyl group,
- at least one nonionic hydrophobic monomer (b) having a polymerizable vinyl group,
- one or several cross-linking monomer(s) (c) including at least one compound of formula (I): 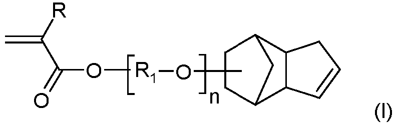 in which:
- R is a hydrogen atom or a methyl group,
- n is equal to 1 and
- R₁ is a C₁-C₂₀ linear or branched alkylene group,
- optionally at least one monomer (d) having a polymerizable vinyl group and an at least C₁₀, preferably C₁₂ to C₃₆, hydrophobic hydrocarbon chain, which is preferably oxyalkylated, distinct from the monomer (b) and
- optionally at least one additional monomer (e) that is optionally nonionic, distinct from the monomer (b),
with the exception of a mixture of monomers comprising 20 parts by weight of methacrylic acid, 10 parts by weight of acrylic acid, 1 part by weight of compound of formula I in which R is a methyl group, n is equal to 1, R₁ is a C₂ linear alkyl group, 20 parts by weight of methyl methacrylate and 39 parts by weight of acrylic acid.

12. The method according to claim 11, further comprising at least the following subsequent step:
- polymerization, in the presence of the polymer P1 obtained previously at the end of the method according to claim 11, of a second mixture of monomers allowing the obtention of a second polymer P2 comprising:
- at least one anionic monomer (a') having a polymerizable vinyl group,
- at least one nonionic hydrophobic monomer (b') having a polymerizable vinyl group,
- one or several cross-linking monomer(s) (c') including at least one compound of formula (I): 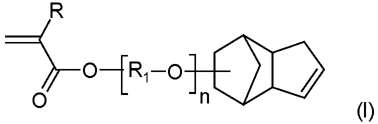 in which:
- R is a hydrogen atom or a methyl group,
- n is equal to 1 and
- R₁ is a C₁-C₂₀ linear or branched alkylene group,
- optionally at least one monomer (d') having a polymerizable vinyl group and an at least C₁₀, preferably C₁₂ to C₃₆, hydrophobic hydrocarbon chain, which is preferably oxyalkylated, distinct from the monomer (b') and
- optionally at least one additional monomer (e') that is optionally nonionic, distinct from the monomer (b').

13. An aqueous composition comprising at least one polymer as defined according to any one of claims 1 to 10 or as obtained according to the method of claim 11 or 12.

14. A use of a monomer of formula (I): 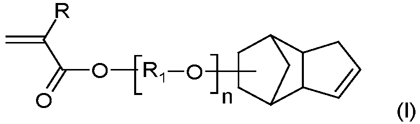 in which:
- R is a hydrogen atom or a methyl group,
- n is equal to 1 and
- R₁ is a C₁-C₂₀ linear or branched alkylene group,
in an amount of less than 5% by weight, based on the total weight of monomers forming the polymer, for the preparation of a polymer, with the exception of a polymer obtained by polymerization of a mixture of monomers comprising 20 parts by weight of methacrylic acid, 10 parts by weight of acrylic acid, 1 part by weight of compound of formula I in which R is a methyl group, n is equal to 1, R₁ is a C₂ linear alkyl group, 20 parts by weight of methyl methacrylate and 39 parts by weight of acrylic acid.
